# EUROPEAN PATENT SPECIFICATION

(11) **EP 3 954 778 B1**
(45) Date of publication and mention of the grant of the patent: **04.10.2023**
(21) Application number: 20190203.8
(22) Date of filing: 10.08.2020
(51) Int. Cl.: C12N 15/52, C12N 9/10, C12P 19/00, C12P 19/04, C12P 19/18

(54) **PRODUCTION OF A MIXTURE OF NEUTRAL NON-FUCOSYLATED OLIGOSACCHARIDES BY A CELL**
HERSTELLUNG EINES GEMISCHS AUS NEUTRALEN, NICHT FUCOSYLIERTEN OLIGOSACCHARIDEN DURCH EINE ZELLE
PRODUCTION D'UN MÉLANGE D'OLIGOSACCHARIDES NON FUCOSYLÉS NEUTRES PAR UNE CELLULE

(43) Date of publication of application: 16.02.2022
(73) Proprietor: Inbiose N.V., 9052 Zwijnaarde (BE)
(72) Inventor: AESAERT, Sofie, 9052 Zwijnaarde (BE); BEAUPREZ, Joeri, 9052 Zwijnaarde (BE); PETERS, Gert, 9052 Zwijnaarde (BE)
(74) Representative: Saelens, Claire

(56) References cited:
- EP-A1- 2 722 394
- PRIEM BERNARD ET AL: "A new fermentation process allows large-scale production of human milk oligosaccharides by metabolically engineered bacteria", GLYCOBIOLOGY, OXFORD UNIVERSITY PRESS, US, vol. 12, no. 4, 1 April 2002 (2002-04-01), pages 235-240, XP002380632, ISSN: 0959-6658, DOI: 10.1093/GLYCOB/12.4.235
- Lars Bode ET AL: "Making Human Milk Oligosaccharides Available for Research and Application - Approaches, Challenges, and Future Opportunities" In: "Prebiotics and Probiotics in Human Milk", 1 January 2017 (2017-01-01), Elsevier, XP055769719, ISBN: 978-0-12-802725-7 pages 251-293, DOI: 10.1016/B978-0-12-802725-7.00009-9,

## Description

The present invention is in the technical field of synthetic biology and metabolic engineering. More particularly, the present invention is in the technical field of fermentation of metabolically engineered cells. The present invention describes a metabolically engineered cell for production of a mixture of at least four different neutral non-fucosylated oligosaccharides. Furthermore, the present invention provides a method for the production of a mixture of at least four different neutral non-fucosylated oligosaccharides by a cell. The purification of at least one of said oligosaccharides from the cultivation is also described.

### Background

Oligosaccharides, often present as glyco-conjugated forms to proteins and lipids, are involved in many vital phenomena such as differentiation, development and biological recognition processes related to the development and progress of fertilization, embryogenesis, inflammation, metastasis and host pathogen adhesion. Oligosaccharides can also be present as unconjugated glycans in body fluids and human milk wherein they also modulate important developmental and immunological processes (Bode, Early Hum. Dev. 1-4 (2015); Reily et al., Nat. Rev. Nephrol. 15, 346-366 (2019); Varki, Glycobiology 27, 3-49 (2017)). There is large scientific and commercial interest in oligosaccharide mixtures due to the wide functional spectrum of oligosaccharides. Yet, the availability of oligosaccharide mixtures is limited as production relies on chemical or chemo-enzymatic synthesis or on purification from natural sources such as e.g. animal milk. Chemical synthesis methods are laborious and time-consuming and because of the large number of steps involved they are difficult to scale-up. Enzymatic approaches using glycosyltransferases offer many advantages above chemical synthesis. Glycosyltransferases catalyze the transfer of a sugar moiety from an activated nucleotide-sugar donor onto saccharide or non-saccharide acceptors (Coutinho et al., J. Mol. Biol. 328 (2003) 307-317). These glycosyltransferases are the source for biotechnologists to synthesize oligosaccharides and are used both in (chemo)enzymatic approaches as well as in cell-based production systems. However, stereospecificity and regioselectivity of glycosyltransferases are still a formidable challenge. In addition, chemo-enzymatic approaches need to regenerate in situ nucleotide-sugar donors. Cellular production of oligosaccharides needs tight control of spatiotemporal availability of adequate levels of nucleotide-sugar donors in proximity of complementary glycosyltransferases. Due to these difficulties, current methods often result in the synthesis of a single oligosaccharide instead of an oligosaccharide mixture.

Priem et al. (Glycobiology 12, p. 235-240 (2002)) describes a recombinant *E. coli* strain which expresses a beta-1,3-N-acetylglucosaminyltransferase (IgtA gene) and a beta-1,4-galactosyltransferase (IgtB gene), both enzymes derived from *Neisseria meningitidis.* Upon administration of lactose, said *E. coli* strain produces lacto-N-neotetraose (LNnT), lacto-N-neohexaose (LNnH) and lacto-N-neooctaose (LNnO). Further, EP2722394 A1 describes a recombinant *Saccharomyces cerevisiae* strain which expresses an enzyme with beta-1,3-N-acetylglucosaminyltransferase activity (*Aspergillus niger* nagA gene) and an enzyme with beta-galactosyltransferase activity (*Streptococcus thermophilus* lacZ gene). Upon administration of lactose and lysing said strain, lacto-N-tetraose and lacto-N-neotetraose are produced. It is an object of the present invention to provide for tools and methods by means of which an oligosaccharide mixture comprising at least four different neutral non-fucosylated oligosaccharides can be produced by a cell in an efficient, time and cost-effective way and if needed, continuous process. According to the invention, this and other objects are achieved by providing a cell and a method for the production of an oligosaccharide mixture comprising at least four different neutral non-fucosylated oligosaccharides wherein the cell is genetically modified for the production of said oligosaccharides.

### Description

Surprisingly, it has now been found that it is possible to produce oligosaccharide mixtures comprising at least four different neutral non-fucosylated oligosaccharides by a single cell. The present invention provides a metabolically engineered cell and a method for the production of an oligosaccharide mixture comprising at least four different neutral non-fucosylated oligosaccharides. The method comprises the steps of providing a cell which expresses at least three glycosyltransferases and is capable to synthesize (a) nucleotide-sugar(s) that is/are donor(s) for said glycosyltransferases cultivating said cell under conditions permissive for producing said oligosaccharide mixture. Preferably, said method comprises separating at least one of said oligosaccharides from the neutral non-fucosylated oligosaccharide mixture. Furthermore, the present invention provides a metabolically engineered cell for production of an oligosaccharide mixture comprising at least four different neutral non-fucosylated oligosaccharides.

### Definitions

The words used in this specification to describe the invention and its various embodiments are to be understood not only in the sense of their commonly defined meanings, but to include by special definition in this specification structure, material or acts beyond the scope of the commonly defined meanings. Thus, if an element can be understood in the context of this specification as including more than one meaning, then its use in a claim must be understood as being generic to all possible meanings supported by the specification and by the word itself.

The various embodiments and aspects of embodiments of the invention disclosed herein are to be understood not only in the order and context specifically described in this specification, but to include any order and any combination thereof. Whenever the context requires, all words used in the singular number shall be deemed to include the plural and vice versa. Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described herein are those well-known and commonly employed in the art. Standard techniques are used for nucleic acid and peptide synthesis. Generally, purification steps are performed according to the manufacturer's specifications.

In the claims which follow, reference characters used to designate claim steps are provided for convenience of description only, and are not intended to imply any particular order for performing the steps.

According to the present invention, the term "polynucleotide(s)" generally refers to any polyribonucleotide or polydeoxyribonucleotide, which may be unmodified RNA or DNA or modified RNA or DNA. "Polynucleotide(s)" include, without limitation, single- and double-stranded DNA, DNA that is a mixture of single- and double-stranded regions or single-, double- and triple-stranded regions, single- and double-stranded RNA, and RNA that is mixture of single- and double-stranded regions, hybrid molecules comprising DNA and RNA that may be single-stranded or, more typically, double-stranded, or triple-stranded regions, or a mixture of single- and double-stranded regions. In addition, "polynucleotide" as used herein refers to triple-stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. As used herein, the term "polynucleotide(s)" also includes DNAs or RNAs as described above that contain one or more modified bases. Thus, DNAs or RNAs with backbones modified for stability or for other reasons are "polynucleotide(s)" according to the present invention. Moreover, DNAs or RNAs comprising unusual bases, such as inosine, or modified bases, such as tritylated bases, are to be understood to be covered by the term "polynucleotides". It will be appreciated that a great variety of modifications have been made to DNA and RNA that serve many useful purposes known to those of skill in the art. The term "polynucleotide(s)" as it is employed herein embraces such chemically, enzymatically or metabolically modified forms of polynucleotides, as well as the chemical forms of DNA and RNA characteristic of viruses and cells, including, for example, simple and complex cells. The term "polynucleotide(s)" also embraces short polynucleotides often referred to as oligonucleotide(s).

"Polypeptide(s)" refers to any peptide or protein comprising two or more amino acids joined to each other by peptide bonds or modified peptide bonds. "Polypeptide(s)" refers to both short chains, commonly referred to as peptides, oligopeptides and oligomers and to longer chains generally referred to as proteins. Polypeptides may contain amino acids other than the 20 gene encoded amino acids. "Polypeptide(s)" include those modified either by natural processes, such as processing and other post-translational modifications, but also by chemical modification techniques. Such modifications are well described in basic texts and in more detailed monographs, as well as in a voluminous research literature, and they are well known to the skilled person. The same type of modification may be present in the same or varying degree at several sites in a given polypeptide. Furthermore, a given polypeptide may contain many types of modifications. Modifications can occur anywhere in a polypeptide, including the peptide backbone, the amino acid sidechains, and the amino or carboxyl termini. Modifications include, for example, acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, lipid attachment, sulfation, gamma-carboxylation of glutamic acid residues, hydroxylation and ADP-ribosylation, selenoylation, transfer-RNA mediated addition of amino acids to proteins, such as arginylation, and ubiquitination. Polypeptides may be branched or cyclic, with or without branching. Cyclic, branched and branched circular polypeptides may result from post-translational natural processes and may be made by entirely synthetic methods, as well.

The term "polynucleotide encoding a polypeptide" as used herein encompasses polynucleotides that include a sequence encoding a polypeptide of the invention. The term also encompasses polynucleotides that include a single continuous region or discontinuous regions encoding the polypeptide (for example, interrupted by integrated phage or an insertion sequence or editing) together with additional regions that also may contain coding and/or non-coding sequences.

"Isolated" means altered "by the hand of man" from its natural state, i.e., if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Similarly, a "synthetic" sequence, as the term is used herein, means any sequence that has been generated synthetically and not directly isolated from a natural source. "Synthesized", as the term is used herein, means any synthetically generated sequence and not directly isolated from a natural source.

The terms "recombinant" or "transgenic" or "metabolically engineered" or "genetically modified", as used herein with reference to a cell or host cell are used interchangeably and indicates that the cell replicates a heterologous nucleic acid, or expresses a peptide or protein encoded by a heterologous nucleic acid (i.e., a sequence "foreign to said cell" or a sequence "foreign to said location or environment in said cell").

Such cells are described to be transformed with at least one heterologous or exogenous gene, or are described to be transformed by the introduction of at least one heterologous or exogenous gene. Metabolically engineered or recombinant or transgenic cells can contain genes that are not found within the native (non-recombinant) form of the cell. Recombinant cells can also contain genes found in the native form of the cell wherein the genes are modified and re-introduced into the cell by artificial means. The terms also encompass cells that contain a nucleic acid endogenous to the cell that has been modified or its expression or activity has been modified without removing the nucleic acid from the cell; such modifications include those obtained by gene replacement, replacement of a promoter; site-specific mutation; and related techniques. Accordingly, a "recombinant polypeptide" is one which has been produced by a recombinant cell. A "heterologous sequence" or a "heterologous nucleic acid", as used herein, is one that originates from a source foreign to the particular cell (e.g. from a different species), or, if from the same source, is modified from its original form or place in the genome. Thus, a heterologous nucleic acid operably linked to a promoter is from a source different from that from which the promoter was derived, or, if from the same source, is modified from its original form or place in the genome. The heterologous sequence may be stably introduced, e.g. by transfection, transformation, conjugation or transduction, into the genome of the host microorganism cell, wherein techniques may be applied which will depend on the cell and the sequence that is to be introduced. Various techniques are known to a person skilled in the art and are, e.g., disclosed in Sambrook et al., Molecular Cloning: A Laboratory Manual, 2nd Ed., Cold Spring Harbor Laboratory Press, Cold Spring Harbor, N.Y. (1989). The term "mutant" cell or microorganism as used within the context of the present invention refers to a cell or microorganism which is genetically modified.

The term "endogenous," within the context of the present invention refers to any polynucleotide, polypeptide or protein sequence which is a natural part of a cell and is occurring at its natural location in the cell chromosome. The term "exogenous" refers to any polynucleotide, polypeptide or protein sequence which originates from outside the cell under study and not a natural part of the cell or which is not occurring at its natural location in the cell chromosome or plasmid.

The term "heterologous" when used in reference to a polynucleotide, gene, nucleic acid, polypeptide, or enzyme refers to a polynucleotide, gene, nucleic acid, polypeptide, or enzyme that is from a source or derived from a source other than the host organism species. In contrast a "homologous" polynucleotide, gene, nucleic acid, polypeptide, or enzyme is used herein to denote a polynucleotide, gene, nucleic acid, polypeptide, or enzyme that is derived from the host organism species. When referring to a gene regulatory sequence or to an auxiliary nucleic acid sequence used for maintaining or manipulating a gene sequence (e.g. a promoter, a 5' untranslated region, 3' untranslated region, poly A addition sequence, intron sequence, splice site, ribosome binding site, internal ribosome entry sequence, genome homology region, recombination site, etc.), "heterologous" means that the regulatory sequence or auxiliary sequence is not naturally associated with the gene with which the regulatory or auxiliary nucleic acid sequence is juxtaposed in a construct, genome, chromosome, or episome. Thus, a promoter operably linked to a gene to which it is not operably linked to in its natural state (i.e. in the genome of a nongenetically engineered organism) is referred to herein as a "heterologous promoter," even though the promoter may be derived from the same species (or, in some cases, the same organism) as the gene to which it is linked.

The term "modified activity" of a protein or an enzyme relates to a change in activity of the protein or the enzyme compared to the wild type, i.e. natural, activity of said protein or enzyme. Said modified activity can either be an abolished, impaired, reduced or delayed activity of said protein or enzyme compared to the wild type activity of the protein or the enzyme but can also be an accelerated or an enhanced activity of said protein or the enzyme compared to the wild type activity of the protein or the enzyme. A modified activity of a protein or an enzyme is obtained by modified expression of said protein or enzyme or is obtained by expression of a modified, i.e. mutant form of the protein or enzyme. A modified activity of an enzyme further relates to a modification in the apparent Michaelis constant Km and/or the apparent maximal velocity (Vmax) of the enzyme.

The term "modified expression" of a gene relates to a change in expression compared to the wild type expression of said gene in any phase of the production process of the encoded protein. Said modified expression is either a lower or higher expression compared to the wild type, wherein the term "higher expression" is also defined as "overexpression" of said gene in the case of an endogenous gene or "expression" in the case of a heterologous gene that is not present in the wild type strain. Lower expression or reduced expression is obtained by means of common well-known technologies for a skilled person (such as the usage of siRNA, CrispR, CrispRi, riboswitches, recombineering, homologous recombination, ssDNA mutagenesis, RNAi, miRNA, asRNA, mutating genes, knocking-out genes, transposon mutagenesis, ...) which are used to change the genes in such a way that they are less-able (i.e. statistically significantly 'less-able' compared to a functional wild-type gene) or completely unable (such as knocked-out genes) to produce functional final products. Next to changing the gene of interest in such a way that lower expression is obtained as described above, lower expression can also be obtained by changing the transcription unit, the promoter, an untranslated region, the ribosome binding site, the Shine Dalgarno sequence or the transcription terminator. Lower expression or reduced expression can for instance be obtained by mutating one or more base pairs in the promoter sequence or changing the promoter sequence fully to a constitutive promoter with a lower expression strength compared to the wild type or an inducible promoter which result in regulated expression or a repressible promoter which results in regulated expression Overexpression or expression is obtained by means of common well-known technologies for a skilled person, wherein said gene is part of an "expression cassette" which relates to any sequence in which a promoter sequence, untranslated region sequence (containing either a ribosome binding sequence or Shine Dalgarno sequence), a coding sequence and optionally a transcription terminator is present, and leading to the expression of a functional active protein. Said expression is either constitutive or regulated.

The term "constitutive expression" is defined as expression that is not regulated by transcription factors other than the subunits of RNA polymerase (e.g. the bacterial sigma factors) under certain growth conditions. Non-limiting examples of such transcription factors are CRP, Lacl, ArcA, Cra, IcIR in E. coli. These transcription factors bind on a specific sequence and may block or enhance expression in certain growth conditions. RNA polymerase binds a specific sequence to initiate transcription, for instance via a sigma factor in prokaryotic hosts.

The term "expression by a natural inducer" is defined as a facultative or regulatory expression of a gene that is only expressed upon a certain natural condition of the host (e.g. organism being in labor, or during lactation), as a response to an environmental change (e.g. including but not limited to hormone, heat, cold, light, oxidative or osmotic stress / signaling), or dependent on the position of the developmental stage or the cell cycle of said host cell including but not limited to apoptosis and autophagy.

The term "control sequences" refers to sequences recognized by the cells transcriptional and translational systems, allowing transcription and translation of a polynucleotide sequence to a polypeptide. Such DNA sequences are thus necessary for the expression of an operably linked coding sequence in a particular cell or organism. Such control sequences can be, but are not limited to, promoter sequences, ribosome binding sequences, Shine Dalgarno sequences, Kozak sequences, transcription terminator sequences. The control sequences that are suitable for prokaryotes, for example, include a promoter, optionally an operator sequence, and a ribosome binding site. Eukaryotic cells are known to utilize promoters, polyadenylation signals, and enhancers. DNA for a presequence or secretory leader may be operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it affects the transcription of the sequence; or a ribosome binding site is operably linked to a coding sequence if it is positioned so as to facilitate translation. Said control sequences can furthermore be controlled with external chemicals, such as, but not limited to, IPTG, arabinose, lactose, allo-lactose, rhamnose or fucose via an inducible promoter or via a genetic circuit that either induces or represses the transcription or translation of said polynucleotide to a polypeptide.

Generally, "operably linked" means that the DNA sequences being linked are contiguous, and, in the case of a secretory leader, contiguous and in reading phase. However, enhancers do not have to be contiguous. The term "glycosyltransferase" as used herein refers to an enzyme capable to catalyze the transfer of sugar moieties from activated donor molecules to specific acceptor molecules, forming glycosidic bonds. The as such synthesized oligosaccharides can be of the linear type or of the branched type and can contain multiple monosaccharide building blocks. A classification of glycosyltransferases using nucleotide diphospho-sugar, nucleotide monophospho-sugar and sugar phosphates and related proteins into distinct sequence-based families has been described (Campbell et al., Biochem. J. 326, 929-939 (1997)) and is available on the CAZy (CArbohydrate-Active EnZymes) website (www.cazy.org).

As used herein the glycosyltransferase can be selected from the list comprising but not limited to: galactosyltransferases, glucosyltransferases, mannosyltransferases, glucosaminyltransferases, N-acetylglucosaminyltransferases, N-acetylgalactosaminyltransferases, N-acetylmannosaminyltransferases, xylosyltransferases, rhamnosyltransferases.

Galactosyltransferases are glycosyltransferases that transfer a galactosyl group (Gal) from an UDP-galactose (UDP-Gal) donor onto a glycan acceptor. Galactosyltransferases comprise beta-1,3-galactosyltransferases, beta-1,4-galactosyltransferases, alpha-1,3-galactosyltransferases and alpha-1,4-galactosyltransferases that transfer a Gal residue from UDP-Gal onto a glycan acceptor via alpha- or beta-glycosidic bonds. Galactosyltransferases can be found but are not limited to the GT2, GT6, GT8, GT25 and GT92 CAZy families. Glucosyltransferases are glycosyltransferases that transfer a glucosyl group (Glc) from an UDP-glucose (UDP-Glc) donor onto a glycan acceptor. Glucosyltransferases comprise alphaglucosyltransferases, beta-1,2-glucosyltransferases, beta-1,3-glucosyltransferases and beta-1,4-glucosyltransferases that transfer a Glc residue from UDP-Glc onto a glycan acceptor via alpha- or beta-glycosidic bonds. Glucosyltransferases can be found but are not limited to the GT1, GT4 and GT25 CAZy families. Mannosyltransferases are glycosyltransferases that transfer a mannose group (Man) from a GDP-mannose (GDP-Man) donor onto a glycan acceptor. Mannosyltransferases comprise alpha-1,2-mannosyltransferases, alpha-1,3-mannosyltransferases and alpha-1,6-mannosyltransferases that transfer a Man residue from GDP-Man onto a glycan acceptor via alpha-glycosidic bonds. Mannosyltransferases can be found but are not limited to the GT22, GT39, GT62 and GT69 CAZy families. N-acetylglucosaminyltransferases are glycosyltransferases that transfer an N-acetylglucosamine group (GlcNAc) from an UDP-N-acetylglucosamine (UDP-GIcNAc) donor onto a glycan acceptor. N-acetylglucosaminyltransferases can be found but are not limited to GT2 and GT4 CAZy families. N-acetylgalactosaminyltransferases are glycosyltransferases that transfer an N-acetylgalactosamine group (GalNAc) from an UDP-N-acetylgalactosamine (UDP-GalNAc) donor onto a glycan acceptor. N-acetylgalactosaminyltransferases can be found but are not limited to GT7, GT12 and GT27 CAZy families. N-acetylmannosaminyltransferases are glycosyltransferases that transfer an N-acetylmannosamine group (ManNAc) from an UDP-N-acetylmannosamine (UDP-ManNAc) donor onto a glycan acceptor. Xylosyltransferases are glycosyltransferases that transfer a xylose residue (Xyl) from an UDP-xylose (UDP-Xyl) donor onto a glycan acceptor. Xylosyltransferases can be found but are not limited to GT61 and GT77 CAZy families. Rhamnosyltransferases are glycosyltransferases that transfer a rhamnose residue from a GDP-rhamnose donor onto a glycan acceptor. Rhamnosyltransferases can be found but are not limited to the GT1, GT2 and GT102 CAZy families.

The terms "nucleotide-sugar" or "activated sugar" are used herein interchangeably and refer to activated forms of monosaccharides. Examples of activated monosaccharides include but are not limited to UDP-galactose (UDP-Gal), UDP-N-acetylglucosamine (UDP-GIcNAc), UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, or UDP-xylose. Nucleotide-sugars act as glycosyl donors in glycosylation reactions. Glycosylation reactions are reactions that are catalyzed by glycosyltransferases.

"Oligosaccharide" as the term is used herein and as generally understood in the state of the art, refers to a saccharide polymer containing a small number, typically three to twenty, of simple sugars, i.e. monosaccharides. The monosaccharides as used herein are reducing sugars. The oligosaccharides can be reducing or non-reducing sugars and have a reducing and a non-reducing end. A reducing sugar is any sugar that is capable of reducing another compound and is oxidized itself, that is, the carbonyl carbon of the sugar is oxidized to a carboxyl group. The oligosaccharide as used in the present invention can be a linear structure or can include branches. The linkage (e.g. glycosidic linkage, galactosidic linkage, glucosidic linkage, etc.) between two sugar units can be expressed, for example, as 1,4, 1->4, or (1-4), used interchangeably herein. Each monosaccharide can be in the cyclic form (e.g. pyranose of furanose form). Linkages between the individual monosaccharide units may include alpha 1->2, alpha 1->3, alpha 1->4, alpha 1->6, alpha 2->1, alpha 2->3, alpha 2->4, alpha 2->6, beta 1->2, beta 1->3, beta 1->4, beta 1->6, beta 2->1, beta 2->3, beta 2->4, and beta 2->6. An oligosaccharide can contain both alpha- and beta-glycosidic bonds or can contain only beta-glycosidic bonds.

The term "monosaccharide" as used herein refers to a sugar that is not decomposable into simpler sugars by hydrolysis, is classed either an aldose or ketose, and contains one or more hydroxyl groups per molecule. Monosaccharides are saccharides containing only one simple sugar. Examples of monosaccharides comprise Hexose, D-Glucopyranose, D-Galactofuranose, D-Galactopyranose, L-Galactopyranose, D-Mannopyranose, D-Allopyranose, L-Altropyranose, D-Gulopyranose, L-Idopyranose, D-Talopyranose, D-Ribofuranose, D-Ribopyranose, D-Arabinofuranose, D-Arabinopyranose, L-Arabinofuranose, L-Arabinopyranose, D-Xylopyranose, D-Lyxopyranose, D-Erythrofuranose, D-Threofuranose, Heptose, L-glycero-D-manno-Heptopyranose (LDmanHep), D-glycero-D-manno-Heptopyranose (DDmanHep), 6-Deoxy-L-altropyranose, 6-Deoxy-D-gulopyranose, 6-Deoxy-D-talopyranose, 6-Deoxy-D-galactopyranose, 6-Deoxy-L-galactopyranose, 6-Deoxy-D-mannopyranose, 6-Deoxy-L-mannopyranose, 6-Deoxy-D-glucopyranose, 2-Deoxy-D-arabino-hexose, 2-Deoxy-D-erythropentose, 2,6-Dideoxy-D-arabino-hexopyranose, 3,6-Dideoxy-D-arabino-hexopyranose, 3,6-Dideoxy-L-arabino-hexopyranose, 3,6-Dideoxy-D-xylo-hexopyranose, 3,6-Dideoxy-D-ribo-hexopyranose, 2,6-Dideoxy-D-ribo-hexopyranose, 3,6-Dideoxy-L-xylo-hexopyranose, 2-Amino-2-deoxy-D-glucopyranose, 2-Amino-2-deoxy-D-galactopyranose, 2-Amino-2-deoxy-D-mannopyranose, 2-Amino-2-deoxy-D-allopyranose, 2-Amino-2-deoxy-L-altropyranose, 2-Amino-2-deoxy-D-gulopyranose, 2-Amino-2-deoxy-L-idopyranose, 2-Amino-2-deoxy-D-talopyranose, 2-Acetamido-2-deoxy-D-glucopyranose, 2-Acetamido-2-deoxy-D-galactopyranose, 2-Acetamido-2-deoxy-D-mannopyranose, 2-Acetamido-2-deoxy-D-allopyranose, 2-Acetamido-2-deoxy-L-altropyranose, 2-Acetamido-2-deoxy-D-gulopyranose, 2-Acetamido-2-deoxy-L-idopyranose, 2-Acetamido-2-deoxy-D-talopyranose, 2-Acetamido-2,6-dideoxy-D-galactopyranose, 2-Acetamido-2,6-dideoxy-L-galactopyranose, 2-Acetamido-2,6-dideoxy-L-mannopyranose, 2-Acetamido-2,6-dideoxy-D-glucopyranose, 2-Acetamido-2,6-dideoxy-L-altropyranose, 2-Acetamido-2,6-dideoxy-D-talopyranose, Erythritol, Arabinitol, Xylitol, Ribitol, Glucitol, Galactitol, Mannitol, D-ribo-Hex-2-ulopyranose, D-arabino-Hex-2-ulofuranose (D-fructofuranose), D-arabino-Hex-2-ulopyranose, L-xylo-Hex-2-ulopyranose, D-lyxo-Hex-2-ulopyranose, D-threo-Pent-2-ulopyranose, D-altro-Hept-2-ulopyranose, 3-C-(Hydroxymethyl)-D-erythofuranose, 2,4,6-Trideoxy-2,4-diamino-D-glucopyranose, 6-Deoxy-3-O-methyl-D-glucose, 3-O-Methyl-D-rhamnose, 2,6-Dideoxy-3-methyl-D-ribohexose, 2-Amino-3-O-[(R)-1-carboxyethyl]-2-deoxy-D-glucopyranose, 2-Acetamido-3-O-[(R)-carboxyethyl]-2-deoxy-D-glucopyranose, 2-Glycolylamido-3-O-[(R)-1-carboxyethyl]-2-deoxy-D-glucopyranose, glucose, galactose, N-acetylglucosamine, glucosamine, mannose, xylose, N-acetylmannosamine, N-acetylgalactosamine, galactosamine, rhamnose, fructose and polyols.

The term "disaccharide" as used herein refers to a saccharide composed of two monosaccharide units. Examples of disaccharides comprise lactose (Gal-b1,4-Glc), lacto-N-biose (Gal-b1,3-GlcNAc), N-acetyllactosamine (Gal-b1,4-GlcNAc), LacDiNAc (GalNAc-b1,4-GlcNAc), N-acetylgalactosaminylglucose (GaINAc-b1,4-Glc).

"Oligosaccharides" as used herein are glycan structures that are composed of three or more monosaccharide subunits that are linked to each other via glycosidic bonds in a linear or in a branched structure. Preferably, the oligosaccharide as described herein contains monosaccharides selected from the list as used herein above. Examples of oligosaccharides include but are not limited to mammalian milk oligosaccharides and human milk oligosaccharides.

As used herein, "mammalian milk oligosaccharide" refers to neutral non-fucosylated mammalian milk oligosaccharides such as but not limited to lacto-N-tetraose, lacto-N-neotetraose, lacto-N-hexaose, lacto-N-neohexaose, para-lacto-N-hexaose and/or galactosylated chitosan.

Mammalian milk oligosaccharides comprise oligosaccharides present in milk found in any phase during lactation including colostrum milk from humans and mammals including but not limited to cows (*Bos Taurus*), sheep (*Ovis aries*), goats (*Capra aegagrus hircus*), bactrian camels (*Camelus bactrianus*), horses (*Equus ferus caballus*), pigs (*Sus scropha*), dogs (*Canis lupus familiaris*), ezo brown bears (*Ursus arctos yesoensis*), polar bear (*Ursus maritimus*), Japanese black bears (*Ursus thibetanus japonicus*), striped skunks (*Mephitis mephitis*), hooded seals (*Cystophora cristata*), Asian elephants (*Elephas maximus*), African elephant (*Loxodonta africana*), giant anteater (*Myrmecophaga tridactyla*), common bottlenose dolphins (*Tursiops truncates*), northern minke whales (*Balaenoptera acutorostrata*), tammar wallabies (*Macropus eugenii*), red kangaroos (*Macropus rufus*), common brushtail possum (*Trichosurus Vulpecula*), koalas (*Phascolarctos cinereus*), eastern quolls (*Dasyurus viverrinus*), platypus (*Ornithorhynchus anatinus*).

A 'fucosylated oligosaccharide' as used herein and as generally understood in the state of the art is an oligosaccharide that is carrying a fucose-residue. Such fucosylated oligosaccharide is a saccharide structure comprising at least three monosaccharide subunits linked to each other via glycosidic bonds, wherein at least one of said monosaccharide subunit is a fucose. A fucosylated oligosaccharide can contain more than one fucose residue, e.g. two, three or more. A fucosylated oligosaccharide can be a neutral oligosaccharide or a charged oligosaccharide e.g. also comprising sialic acid structures. Fucose can be linked to other monosaccharide subunits comprising glucose, galactose, GlcNAc via alpha-glycosidic bonds comprising alpha-1,2 alpha-1,3, alpha-1,4, alpha-1,6 linkages.

Examples comprise 2'-fucosyllactose (2'FL), 3-fucosyllactose (3FL), 4-fucosyllactose (4FL), 6-fucosyllactose (6FL), difucosyllactose (diFL), lactodifucotetraose (LDFT), Lacto-N-fucopentaose I (LNFP I), Lacto-N-fucopentaose II (LNFP II), Lacto-N-fucopentaose III (LNFP III), lacto-N-fucopentaose V (LNFP V), lacto-N-fucopentaose VI (LNFP VI), lacto-N-neofucopentaose I, lacto-N-difucohexaose I (LDFH I), lacto-N-difucohexaose II (LDFH II), Monofucosyllacto-N-hexaose III (MFLNH III), Difucosyllacto-N-hexaose (DFLNHa), difucosyl-lacto-N-neohexaose, 3'-sialyl-3-fucosyllactose, disialomonofucosyllacto-N-neohexaose, monofucosylmonosialyllacto-N-octaose (sialyl Lea), sialyllacto-N-fucohexaose II, disialyllacto-N-fucopentaose II, monofucosyldisialyllacto-N-tetraose.

A 'neutral oligosaccharide' as used herein and as generally understood in the state of the art is an oligosaccharide that has no negative charge originating from a carboxylic acid group. Examples of such neutral oligosaccharide comprise 2'-fucosyllactose (2'FL), 3-fucosyllactose (3FL), 2', 3-difucosyllactose (diFL), lacto-N-triose II, lacto-N-tetraose, lacto-N-neotetraose, lacto-N-fucopentaose I, lacto-N-neofucopentaose I, lacto-N-fucopentaose II, lacto-N-fucopentaose III, lacto-N-fucopentaose V, lacto-N-fucopentaose VI, lacto-N-neofucopentaose V, lacto-N-difucohexaose I, lacto-N-difucohexaose II, 6'-galactosyllactose, 3'-galactosyllactose, lacto-N-hexaose, lacto-N-neohexaose, para-lacto-N-hexaose, para-lacto-N-neohexaose, difucosyl-lacto-N-hexaose and difucosyl-lacto-N-neohexaose.

'A neutral non-fucosylated oligosaccharide' as used herein and as generally understood in the state of the art is an oligosaccharide that has no negative charge originating from a carboxylic acid group and does not contain a fucose residue. Examples of such neutral non-fucosylated oligosaccharides comprise lacto-N-triose II, lacto-N-tetraose, lacto-N-neotetraose, 6'-galactosyllactose, 3'-galactosyllactose, lacto-N-hexaose, lacto-N-neohexaose, para-lacto-N-hexaose and para-lacto-N-neohexaose.

A 'galactosylation pathway' as used herein is a biochemical pathway consisting of the enzymes and their respective genes, galactose-1-epimerase, galactokinase, glucokinase, galactose-1-phosphate uridylyltransferase, UDP-glucose 4-epimerase, glucose-1-phosphate uridylyltransferase, and/or glucophosphomutase, combined with a galactosyltransferase leading to an alpha or beta bound galactose on the 2, 3, 4, and/or 6 hydroxyl group of an oligosaccharide.

An 'N-acetylglucosamine carbohydrate pathway' as used herein is a biochemical pathway consisting of the enzymes and their respective genes, L-glutamine-D-fructose-6-phosphate aminotransferase, glucosamine-6-phosphate deaminase, phosphoglucosamine mutase, N-acetylglucosamine-6-phosphate deacetylase, glucosamine 6-phosphate N-acetyltransferase, N-acetylglucosamine-1-phosphate uridylyltransferase, glucosamine-1-phosphate acetyltransferase, and/or glucosamine-1-phosphate acetyltransferase, combined with a glycosyltransferase leading to an alpha or beta bound N-acetylglucosamine on the 3, 4, and/or 6 hydroxylgroup of an oligosaccharide.

The term "purified" refers to material that is substantially or essentially free from components which interfere with the activity of the biological molecule. For cells, saccharides, nucleic acids, and polypeptides, the term "purified" refers to material that is substantially or essentially free from components which normally accompany the material as found in its native state. Typically, purified saccharides, oligosaccharides, proteins or nucleic acids of the invention are at least about 50 %, 55 %, 60 %, 65 %, 70 %, 75 %, 80 % or 85 % pure, usually at least about 90 %, 91 %, 92 %, 93 %, 94 %, 95 %, 96 %, 97 %, 98 %, or 99 % pure as measured by band intensity on a silver stained gel or other method for determining purity. Purity or homogeneity can be indicated by a number of means well known in the art, such as polyacrylamide gel electrophoresis of a protein or nucleic acid sample, followed by visualization upon staining. For certain purposes high resolution will be needed and HPLC or a similar means for purification utilized. For oligosaccharides, purity can be determined using methods such as but not limited to thin layer chromatography, gas chromatography, NMR, HPLC, capillary electrophoresis or mass spectroscopy.

The term "cultivation" refers to the culture medium wherein the cell is cultivated or fermented, the cell itself, and the oligosaccharides that are produced by the cell in whole broth, i.e. inside (intracellularly) as well as outside (extracellularly) of the cell.

The terms "precursor" as used herein refers to substances which are taken up or synthetized by the cell for the specific production of a neutral non-fucosylated oligosaccharide according to the present invention. In this sense a precursor can be an acceptor as defined herein, but can also be another substance, metabolite, which is first modified within the cell as part of the biochemical synthesis route of the oligosaccharide. Examples of such precursors comprise the acceptors as defined herein, and/or glucose, galactose, fructose, glycerol, mannose, maltose, sucrose, lactose, glucose-1-phosphate, galactose-1-phosphate, UDP-glucose, UDP-galactose, glucose-6-phosphate, fructose-6-phosphate, fructose-1,6-bisphosphate, glycerol-3-phosphate, dihydroxyacetone, glyceraldehyde-3-phosphate, dihydroxyacetone-phosphate, glucosamine-6-phosphate, glucosamine, N-acetyl-glucosamine-6-phosphate, N-acetyl-glucosamine, N-acetyl-mannosamine, N-acetylmannosamine-6-phosphate, UDP-N-acetylglucosamine, N-acetylglucosamine-1-phosphate, mannose-6-phosphate, mannose-1-phosphate, GDP-mannose and/or GDP-4-dehydro-6-deoxy-α-D-mannose.

Optionally, the cell is transformed to comprise at least one nucleic acid sequence encoding a protein selected from the group consisting of lactose transporter, transporter for a nucleotide-activated sugar wherein said transporter internalizes a to the medium added precursor for neutral non-fucosylated oligosaccharide synthesis.

The term "acceptor" as used herein refers to di- or oligosaccharides which can be modified by a glycosyltransferase. Examples of such acceptors comprise lactose, lacto-N-biose (LNB), lacto-N-triose, lacto-N-tetraose (LNT), lacto-N-neotetraose (LNnT), N-acetyl-lactosamine (LacNAc), lacto-N-pentaose (LNP), lacto-N-neopentaose, para lacto-N-pentaose, para lacto-N-neopentaose, lacto-N-novopentaose I, lacto-N-hexaose (LNH), lacto-N-neohexaose (LNnH), para lacto-N-neohexaose (pLNnH), para lacto-N-hexaose (pLNH), lacto-N-heptaose, lacto-N-neoheptaose, para lacto-N-neoheptaose, para lacto-N-heptaose, lacto-N-octaose (LNO), lacto-N-neooctaose, iso lacto-N-octaose, para lacto-N-octaose, iso lacto-N-neooctaose, novo lacto-N-neooctaose, para lacto-N-neooctaose, iso lacto-N-nonaose, novo lacto-N-nonaose, lacto-N-nonaose, lacto-N-decaose, iso lacto-N-decaose, novo lacto-N-decaose, lacto-N-neodecaose, galactosyllactose, a lactose extended with 1, 2, 3, 4, 5, or a multiple of N-acetyllactosamine units and/or 1, 2, 3, 4, 5, or a multiple of, Lacto-N-biose units, and oligosaccharide containing 1 or multiple N-acetyllactosamine units and or 1 or multiple lacto-N-biose units or an intermediate into oligosaccharide versions thereof.

### Detailed description of the invention

The invention is set out in the appended set of claims. According to a first embodiment, the present invention provides a metabolically engineered cell for the production of a mixture comprising at least four different neutral non-fucosylated oligosaccharides. Herein, a single metabolically engineered cell is provided which expresses at least three glycosyltransferases and is capable of synthesizing one or more sugar-nucleotide(s) which is/are donor(s) for said glycosyltransferases.

According to second embodiment, the present invention provides a method for the production of a mixture comprising at least four different neutral non-fucosylated oligosaccharides. The method comprises the steps of:
i) providing a cell that expresses at least three glycosyltransferases and is capable to synthesize one or more nucleotide-sugar(s), wherein said nucleotide-sugar(s) is/are donor(s) for said glycosyltransferases, and
ii) cultivating said cell under conditions permissive to express said glycosyltransferases and to synthesize said nucleotide-sugar(s),
iii) preferably, separating at least one of said neutral non-fucosylated oligosaccharides from the cultivation.

According to the invention, said method for the production of a mixture comprising at least four different neutral non-fucosylated oligosaccharides can make use of a non-metabolically engineered cell or can make use of a metabolically engineered cell as disclosed herein.

In the scope of the present invention, permissive conditions are understood to be conditions relating to physical or chemical parameters including but not limited to temperature, pH, pressure, osmotic pressure and product/educt concentration.

In a particular embodiment, the permissive conditions may include a temperature-range of 30 +/- 20 degrees centigrade, a pH-range of 7 +/- 3.

In another preferred embodiment of the method of the invention, the cultivation is fed with a precursor for the synthesis of any one of said neutral non-fucosylated oligosaccharides in said mixture. In a further preferred embodiment of the method, the cultivation is fed with at least two precursors for the synthesis of any one or more of said neutral non-fucosylated oligosaccharides in the mixture. This can be useful if two or more glycosyltransferases of the same classification are used which have a different affinity for the production of a mixture of neutral non-fucosylated oligosaccharides according to the present invention. The present invention provides different types of cells for said production of an oligosaccharide mixture comprising four or more neutral non-fucosylated oligosaccharides with a single metabolically engineered cell. For example, the present invention provides a cell wherein said cell expresses three different glycosyltransferases and said cell synthesizes one single nucleotide-sugar which is donor for all of said three expressed glycosyltransferases. The present invention also provides a cell wherein said cell expresses three or more glycosyltransferases and said cell synthesizes one or more different nucleotide-sugar(s) which is/are donor(s) for all of said expressed glycosyltransferases.

According to a preferred embodiment of the method and cell according to the invention, the metabolically engineered cell is modified with gene expression modules wherein the expression from any one of said expression modules is constitutive or is created by a natural inducer.

Said expression modules are also known as transcriptional units and comprise polynucleotides for expression of recombinant genes including coding gene sequences and appropriate transcriptional and/or translational control signals that are operably linked to the coding genes. Said control signals comprise promoter sequences, untranslated regions, ribosome binding sites, terminator sequences. Said expression modules can contain elements for expression of one single recombinant gene but can also contain elements for expression of more recombinant genes or can be organized in an operon structure for integrated expression of two or more recombinant genes. Said polynucleotides may be produced by recombinant DNA technology using techniques well-known in the art. Methods which are well known to those skilled in the art to construct expression modules include, for example, in vitro recombinant DNA techniques, synthetic techniques, and in vivo genetic recombination. See, for example, the techniques described in Sambrook et al. (2001) Molecular Cloning: a laboratory manual, 3rd Edition, Cold Spring Harbor Laboratory Press, CSH, New York or to Current Protocols in Molecular Biology, John Wiley and Sons, N.Y. (1989 and yearly updates).

According to a preferred embodiment of the present invention, the cell is modified with one or more expression modules. The expression modules can be integrated in the genome of said cell or can be presented to said cell on a vector. Said vector can be present in the form of a plasmid, cosmid, phage, liposome, or virus, which is to be stably transformed/transfected into said metabolically engineered cell. Such vectors include, among others, chromosomal, episomal and virus-derived vectors, e.g., vectors derived from bacterial plasmids, from bacteriophage, from transposons, from yeast episomes, from insertion elements, from yeast chromosomal elements, from viruses, and vectors derived from combinations thereof, such as those derived from plasmid and bacteriophage genetic elements, such as cosmids and phagemids. These vectors may contain selection markers such as but not limited to antibiotic markers, auxotrophic markers, toxin-antitoxin markers, RNA sense/antisense markers. The expression system constructs may contain control regions that regulate as well as engender expression. Generally, any system or vector suitable to maintain, propagate or express polynucleotides and/or to express a polypeptide in a host may be used for expression in this regard. The appropriate DNA sequence may be inserted into the expression system by any of a variety of well-known and routine techniques, such as, for example, those set forth in Sambrook et al., see above. For recombinant production, cells can be genetically engineered to incorporate expression systems or portions thereof or polynucleotides of the invention. Introduction of a polynucleotide into the cell can be effected by methods described in many standard laboratory manuals, such as Davis et al., Basic Methods in Molecular Biology, (1986), and Sambrook et al., 1989, supra.

As used herein an expression module comprises polynucleotides for expression of at least one recombinant gene. Said recombinant gene is involved in the expression of a polypeptide acting in the synthesis of said neutral non-fucosylated oligosaccharide mixture; or said recombinant gene is linked to other pathways in said host cell that are not involved in the synthesis of said mixture of three or more neutral non-fucosylated oligosaccharides. Said recombinant genes encode endogenous proteins with a modified expression or activity, preferably said endogenous proteins are overexpressed; or said recombinant genes encode heterologous proteins that are heterogeneously introduced and expressed in said modified cell, preferably overexpressed. The endogenous proteins can have a modified expression in the cell which also expresses a heterologous protein.

According to a preferred embodiment of the present invention, the expression of each of said expression modules is constitutive or created by a natural inducer. As used herein, constitutive expression should be understood as expression of a gene that is transcribed continuously in an organism. Expression that is created by a natural inducer should be understood as a facultative or regulatory expression of a gene that is only expressed upon a certain natural condition of the host (e.g. organism being in labor, or during lactation), as a response to an environmental change (e.g. including but not limited to hormone, heat, cold, light, oxidative or osmotic stress / signaling), or dependent on the position of the developmental stage or the cell cycle of said host cell including but not limited to apoptosis and autophagy.

In an embodiment of the method and/or cell according to the invention, the oligosaccharide mixture is composed of neutral non-fucosylated oligosaccharides. Neutral oligosaccharides are oligosaccharide structures that do not contain a negatively charged monosaccharide subunit including N-acetylneuraminic acid (Neu5Ac), commonly known as sialic acid, N-glycolylneuraminic acid (Neu5Gc), glucuronate and galacturonate. Neutral oligosaccharides are also referred to as non-acidic oligosaccharides. Sialic acid belongs to the family of derivatives of neuraminic acid (5-amino-3,5-dideoxy-D-glycero-D-galacto-non-2-ulosonic acid). Neu5Gc is a derivative of sialic acid, which is formed by hydroxylation of the N-acetyl group at C5 of Neu5Ac. Neutral oligosaccharides are non-sialylated oligosaccharides, and thus do not contain an acidic monosaccharide subunit. Neutral non-fucosylated oligosaccharides are non-charged non-fucosylated oligosaccharides that lack any fucose subunit.

In an embodiment of the method and/or cell according to the invention, the oligosaccharide mixture comprises at least three different neutral non-fucosylated oligosaccharides differing in degree of polymerization (DP). The degree of polymerization of an oligosaccharide refers to the number of monosaccharide units present in the oligosaccharide structure. As used herein, the degree of polymerization of an oligosaccharide is three (DP3) or more, the latter comprising any one of 4 (DP4), 5 (DP5), 6 (DP6) or longer. The oligosaccharide mixture as described herein preferably comprise at least three different neutral non-fucosylated oligosaccharides differing in degree of polymerization from each other.

According to one embodiment of the method and/or cell of the invention, the cell produces a mixture comprising four different neutral non-fucosylated oligosaccharides or more than four different neutral non-fucosylated oligosaccharides. In one embodiment, such mixture comprises at least four different oligosaccharides wherein three of the oligosaccharides have a different degree of polymerization. In one embodiment, all of said oligosaccharides in the mixture have a different degree of polymerization as described herein.

In an embodiment of the method and/or cell according to the invention, any one of said glycosyltransferases is chosen from the list comprising galactosyltransferases, glucosyltransferases, mannosyltransferases, N-acetylglucosaminyltransferases, N-acetylgalactosaminyltransferases, N-acetylmannosaminyltransferases, xylosyltransferases, glucosaminyltransferases, rhamnosyltransferases and xylosyltransferases as defined herein.

In a further aspect of the method and/or cell of the invention, the cell is modified in the expression or activity of at least one of said glycosyltransferases. In a preferred embodiment, said glycosyltransferase is an endogenous protein of the cell with a modified expression or activity, preferably said endogenous glycosyltransferase is overexpressed; alternatively said glycosyltransferase is an heterologous protein that is heterogeneously introduced and expressed in said cell, preferably overexpressed. Said endogenous glycosyltransferase can have a modified expression in the cell which also expresses a heterologous glycosyltransferase.

In another aspect of the method and/or cell of the invention, at least one of said glycosyltransferases is an N-acetylglucosaminyltransferase and the cell is capable to synthesize UDP-GIcNAc. The UDP-GIcNAc can be provided by an enzyme expressed in the cell or by the metabolism of the cell. Such cell producing an UDP-GIcNAc can express enzymes converting, e.g. GlcNAc, which is to be added to the cell, to UDP-GIcNAc. These enzymes may be an N-acetyl-D-glucosamine kinase, an N-acetylglucosamine-6-phosphate deacetylase, a phosphoglucosamine mutase, and an N-acetylglucosamine-1-phosphate uridyltransferase/glucosamine-1-phosphate acetyltransferase from several species including *Homo sapiens, Escherichia coli.* Preferably, the cell is modified to produce UDP-GIcNAc. More preferably, the cell is modified for enhanced UDP-GIcNAc production. Said modification can be any one or more chosen from the group comprising knock-out of an N-acetylglucosamine-6-phosphate deacetylase, over-expression of an L-glutamine-D-fructose-6-phosphate aminotransferase, over-expression of a a phosphoglucosamine mutase, and over-expression of an N-acetylglucosamine-1-phosphate uridyltransferase/glucosamine-1-phosphate acetyltransferase.

In another aspect of the method and/or cell of the invention, at least one of said glycosyltransferases is a galactosyltransferase and the cell is capable to synthesize UDP-Gal. The UDP-Gal can be provided by an enzyme expressed in the cell or by the metabolism of the cell. Such cell producing UDP-Gal can express an enzyme converting, e.g. UDP-glucose, to UDP-Gal. This enzyme may be, e.g., the UDP-glucose-4-epimerase GalE like as known from several species including *Homo sapiens, Escherichia coli,* and *Rattus norvegicus.* Preferably, the cell is modified to produce UDP-Gal. More preferably, the cell is modified for enhanced UDP-Gal production. Said modification can be any one or more chosen from the group comprising knock-out of an bifunctional 5'-nucleotidase/UDP-sugar hydrolase encoding gene, knock-out of a galactose-1-phosphate uridylyltransferase encoding gene and over-expression of an UDP-glucose-4-epimerase encoding gene.

In another embodiment of the method and/or cell of the invention, the cell is capable to synthesize any one of said nucleotide-sugars chosen from the list comprising UDP-GIcNAc, UDP-Gal, UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, UDP-xylose. In a preferred embodiment, said cell is metabolically engineered for the production of a nucleotide-sugar. In another preferred embodiment, the cell is modified and/or engineered for the optimized production of a nucleotide-sugar.

According to the method and/or cell of the invention, at least one of said neutral non-fucosylated oligosaccharides of said mixture is galactosylated, glucosylated, xylosylated, mannosylated, contains an N-acetylglucosamine, contains an N-acetylgalactosamine, contains a rhamnose and/or contains an N-acetylmannosamine.

Preferably, the mixture of neutral non-fucosylated oligosaccharides comprises at least one oligosaccharide of 3 or more monosaccharide subunits linked to each other via glycosidic bonds, wherein at least one of said monosaccharide residues is a GlcNAc residue. Said oligosaccharide can contain more than one GlcNAc residue, e.g. two, three or more. GlcNAc can be present at the reducing end of the oligosaccharide. Said GlcNAc can also be present at the non-reducing end of said oligosaccharide. Said GlcNAc can also be present within the oligosaccharide structure. GlcNAc can be linked to other monosaccharide subunits like e.g. galactose.

Alternatively or additionally, the neutral non-fucosylated oligosaccharide mixture comprises at least one galactosylated oligosaccharide and contains at least one galactose monosaccharide subunit. Said galactosylated oligosaccharide is a saccharide structure comprising at least three monosaccharide subunits linked to each other via glycosidic bonds, wherein at least one of said monosaccharide subunit is a galactose. Said galactosylated oligosaccharide can contain more than one galactose residue, e.g. two, three or more. Galactose can be linked to other monosaccharide subunits comprising glucose and GlcNAc. In a preferred embodiment of the method and/or cell of the invention, any one of said neutral non-fucosylated oligosaccharides is translocated to the outside of the cell by a passive transport i.e. without means of an active transport system consuming energy from said cell.

In another preferred embodiment of the method and/or cell of the invention, the cell is further metabolically engineered for
i) modified expression of an endogenous membrane protein, and/or
ii) modified activity of an endogenous membrane protein, and/or
iii) expression of a homologous membrane protein, and/or
iv) expression of a heterologous membrane protein,
wherein said membrane protein is involved in the secretion of any one of said neutral non-fucosylated oligosaccharides outside said cell. Said cell can express one of said membrane proteins that is involved in the secretion of any one of said neutral non-fucosylated oligosaccharides from said cell to the outside of said cell. Said cell can also express more than one of said membrane proteins. Any one of said membrane proteins can translocate one or more of said neutral non-fucosylated oligosaccharides to the outside of said cell. Said cell producing a mixture of at least four neutral non-fucosylated oligosaccharides can translocate any one of said oligosaccharides comprising passive diffusion, channel membrane proteins, membrane transporter proteins, membrane carrier proteins.

In another preferred embodiment of the method and/or cell of the invention, the cell is further metabolically engineered for
i) modified expression of an endogenous membrane protein, and/or
ii) modified activity of an endogenous membrane protein, and/or
iii) expression of a homologous membrane protein, and/or
iv) expression of a heterologous membrane protein,
wherein said membrane protein is involved in the uptake of a precursor for the synthesis of any one of said neutral non-fucosylated oligosaccharides. The term "precursor" should be understood as explained in the definitions as disclosed herein. Said cell can express one of said membrane proteins that is involved in the uptake of any type of precursor used in the synthesis of any one of said neutral non-fucosylated oligosaccharides. Said cell can also express more than one of said membrane proteins, involved in the uptake of at least one of said precursors. Said cell can be modified for the uptake of more than one precursor for the synthesis of any one of said neutral non-fucosylated oligosaccharides.

In another embodiment of the method and/or cell, the cell is producing a precursor for the synthesis of any one of said neutral non-fucosylated oligosaccharides. In a preferred embodiment, said cell is producing one or more precursors for the synthesis of said neutral non-fucosylated oligosaccharide mixture. In a more preferred embodiment, said cell is modified for optimized production of any one of said precursors for the synthesis of any one of said neutral non-fucosylated oligosaccharides.

In a preferred embodiment, the present invention provides a method for the production of a mixture of at least four neutral non-fucosylated oligosaccharides with a cell wherein said cell completely converts any one of said precursors into any one of said oligosaccharides.

In a preferred embodiment of the method and/or cell of the invention, the cell confers enhanced bacteriophage resistance. Said enhancement of bacteriophage resistance can be derived from reduced expression of an endogenous membrane protein and/or mutation of an endogenous membrane protein encoding gene. The term "phage insensitive" or "phage resistant" or "phage resistance" or "phage resistant profile" is understood to mean a bacterial strain that is less sensitive, and preferably insensitive to infection and/ or killing by phage and/ or growth inhibition. As used herein, the terms "anti-phage activity" or "resistant to infection by at least one phage" refers to an increase in resistance of a bacterial cell expressing a functional phage resistance system to infection by at least one phage family in comparison to a bacterial cell of the same species under the same developmental stage (e.g. culture state) which does not express a functional phage resistance system, as may be determined by e.g. bacterial viability, phage lysogeny, phage genomic replication and phage genomic degradation. The phage can be a lytic phage or a temperate (lysogenic) phage. Membrane proteins involved in bacteriophage resistance of a cell comprise OmpA, OmpC, OmpF, OmpT, BtuB, TolC, LamB, FhuA, TonB, FadL, Tsx, FepA, YncD, PhoE, and NfrA and homologs thereof.

In a preferred embodiment of the method and/or cell of the invention, the cell confers reduced viscosity. Reduced viscosity of a cell can be obtained by a modified cell wall biosynthesis. Cell wall biosynthesis can be modified comprising reduced or abolished synthesis of for example poly-N-acetyl-glucosamine, the enterobacterial common antigen, cellulose, colanic acid, core oligosaccharides, osmoregulated periplasmic glucans and glucosylglycerol, glycan, and trehalose.

In another embodiment of the method and/or cell of the invention, at least one of the neutral non-fucosylated oligosaccharides produced by said cell is a mammalian milk oligosaccharide. Said cell can produce one mammalian milk oligosaccharide in said produced mixture of at least four neutral non-fucosylated oligosaccharides. Said cell can produce more than one mammalian milk oligosaccharide in said produced mixture of at least four neutral non-fucosylated oligosaccharides. In a preferred embodiment, all said oligosaccharides in the produced mixture of at least four neutral non-fucosylated oligosaccharides are mammalian milk oligosaccharides.

According to the present invention, the method as described herein preferably comprises a step of separating at least one of said neutral non-fucosylated oligosaccharides from said cultivation.

The terms "separating from said cultivation" means harvesting, collecting, or retrieving any of said oligosaccharides from the cell and/or the medium of its growth.

Any one of said neutral non-fucosylated oligosaccharides can be separated in a conventional manner from the aqueous culture medium, in which the cell was grown. In case said oligosaccharide is still present in the cells producing the neutral non-fucosylated oligosaccharide mixture, conventional manners to free or to extract said oligosaccharide out of the cells can be used, such as cell destruction using high pH, heat shock, sonication, French press, homogenization, enzymatic hydrolysis, chemical hydrolysis, solvent hydrolysis, detergent, hydrolysis,... The culture medium and/or cell extract together and separately can then be further used for separating said oligosaccharide. This preferably involves clarifying said oligosaccharide containing mixture to remove suspended particulates and contaminants, particularly cells, cell components, insoluble metabolites and debris produced by culturing the genetically modified cell. In this step, said oligosaccharide containing mixture can be clarified in a conventional manner.

Preferably, said oligosaccharide containing mixture is clarified by centrifugation, flocculation, decantation and/or filtration. A second step of separating said oligosaccharide from said oligosaccharide containing mixture preferably involves removing substantially all the proteins, as well as peptides, amino acids, RNA and DNA and any endotoxins and glycolipids that could interfere with the subsequent separation step, from said oligosaccharide containing mixture, preferably after it has been clarified. In this step, proteins and related impurities can be removed from said oligosaccharide containing mixture in a conventional manner. Preferably, proteins, salts, by-products, colour and other related impurities are removed from said oligosaccharide containing mixture by ultrafiltration, nanofiltration, reverse osmosis, microfiltration, activated charcoal or carbon treatment, tangential flow high-performance filtration, tangential flow ultrafiltration, affinity chromatography, ion exchange chromatography (such as but not limited to cation exchange, anion exchange, mixed bed ion exchange), hydrophobic interaction chromatography and/or gel filtration (i.e., size exclusion chromatography), particularly by chromatography, more particularly by ion exchange chromatography or hydrophobic interaction chromatography or ligand exchange chromatography. With the exception of size exclusion chromatography, proteins and related impurities are retained by a chromatography medium or a selected membrane, said oligosaccharide remains in the said oligosaccharide containing mixture.

Another aspect of the invention provides for a method and a cell wherein a mixture comprising at least four different neutral non-fucosylated oligosaccharides is produced in and/or by a fungal, yeast, bacterial, insect, animal and plant expression system or cell as described herein. The expression system or cell is chosen from the list comprising a bacterium, a yeast, or a fungus, or, refers to a plant or animal cell. The latter bacterium preferably belongs to the phylum of the Proteobacteria or the phylum of the Firmicutes or the phylum of the Cyanobacteria or the phylum Deinococcus-Thermus. The latter bacterium belonging to the phylum Proteobacteria belongs preferably to the family Enterobacteriaceae, preferably to the species *Escherichia coli.* The latter bacterium preferably relates to any strain belonging to the species *Escherichia coli* such as but not limited to *Escherichia coli* B, *Escherichia coli* C, *Escherichia coli* W, *Escherichia coli* K12, *Escherichia coli* Nissle. More specifically, the latter term relates to cultivated *Escherichia coli* strains - designated as *E. coli* K12 strains - which are well-adapted to the laboratory environment, and, unlike wild type strains, have lost their ability to thrive in the intestine. Well-known examples of the *E. coli* K12 strains are K12 Wild type, W3110, MG1655, M182, MC1000, MC1060, MC1061, MC4100, JM101, NZN111 and AA200. Hence, the present invention specifically relates to a mutated and/or transformed *Escherichia coli* cell or strain as indicated above wherein said *E. coli* strain is a K12 strain. More preferably, the *Escherichia coli* K12 strain is *E. coli* MG1655. The latter bacterium belonging to the phylum Firmicutes belongs preferably to the Bacilli, preferably Lactobacilliales, with members such as *Lactobacillus lactis, Leuconostoc mesenteroides,* or Bacillales with members such as from the genus Bacillus, such as *Bacillus subtilis* or, *B. amyloliquefaciens.* The latter Bacterium belonging to the phylum Actinobacteria, preferably belonging to the family of the Corynebacteriaceae, with members *Corynebacterium glutamicum* or *C*. *afermentans,* or belonging to the family of the Streptomycetaceae with members *Streptomyces griseus* or *S. fradiae.* The latter yeast preferably belongs to the phylum of the Ascomycota or the phylum of the Basidiomycota or the phylum of the Deuteromycota or the phylum of the Zygomycetes. The latter yeast belongs preferably to the genus Saccharomyces, Pichia, Komagataella, Hansenula, Kluyveromyces, Yarrowia or Starmerella. The latter fungus belongs preferably to the genus Rhizopus, Dictyostelium, Penicillium, Mucor or Aspergillus.

The microorganism or cell as used herein is capable to grow on a monosaccharide, disaccharide, oligosaccharide, polysaccharide, polyol, a complex medium or a mixture thereof as the main carbon source. With the term main is meant the most important carbon source for the bioproducts of interest, biomass formation, carbon dioxide and/or by-products formation (such as acids and/or alcohols, such as acetate, lactate, and/or ethanol), i.e. 20, 30, 40, 50, 60, 70, 75, 80, 85, 90, 95, 98, 99 % of all the required carbon is derived from the above-indicated carbon source. In one embodiment of the invention, said carbon source is the sole carbon source for said organism, i.e. 100 % of all the required carbon is derived from the above-indicated carbon source. Common main carbon sources comprise but are not limited to glucose, glycerol, fructose, maltose, lactose, arabinose, malto-oligosaccharides, maltotriose, sorbitol, xylose, rhamnose, sucrose, galactose, mannose, methanol, ethanol, trehalose, starch, cellulose, hemicellulose, corn-steep liquor, high-fructose syrup, acetate, citrate, lactate and pyruvate. With the term complex medium is meant a medium for which the exact constitution is not determined. Examples are molasses, corn steep liquor, peptone, tryptone or yeast extract.

In a further preferred embodiment, the microorganism or cell described herein is using a split metabolism having a production pathway and a biomass pathway as described in WO2012/007481. Said organism can, for example, be genetically modified to accumulate fructose-6-phosphate by altering the genes selected from the phosphoglucoisomerase gene, phosphofructokinase gene, fructose-6-phosphate aldolase gene, fructose isomerase gene, and/or fructose:PEP phosphotransferase gene.

In a further preferred embodiment, the methods as described herein also provide for a further purification of any one or more of said neutral non-fucosylated oligosaccharide(s) from the oligosaccharide mixture. A further purification of said oligosaccharide(s) may be accomplished, for example, by use of (activated) charcoal or carbon, nanofiltration, ultrafiltration or ion exchange to remove any remaining DNA, protein, LPS, endotoxins, or other impurity. Alcohols, such as ethanol, and aqueous alcohol mixtures can also be used. Another purification step is accomplished by crystallization, evaporation or precipitation of the product. Another purification step is to dry, spray dry or lyophilize the produced neutral non-fucosylated oligosaccharide.

In an exemplary embodiment, the separation and purification of at least one of the produced neutral non-fucosylated oligosaccharides is made in a process, comprising the following steps in any order:
a) contacting the cultivation or a clarified version thereof with a nanofiltration membrane with a molecular weight cut-off (MWCO) of 600-3500 Da ensuring the retention of produced oligosaccharide(s) and allowing at least a part of the proteins, salts, by-products, colour and other related impurities to pass,
b) conducting a diafiltration process on the retentate from step a), using said membrane, with an aqueous solution of an inorganic electrolyte, followed by optional diafiltration with pure water to remove excess of the electrolyte,
c) and collecting the retentate enriched in the oligosaccharide(s) in the form of a salt from the cation of said electrolyte.

In an alternative exemplary embodiment, the separation and purification of at least one of the produced neutral non-fucosylated oligosaccharides is made in a process, comprising the following steps in any order: subjecting the cultivation or a clarified version thereof to two membrane filtration steps using different membranes, wherein
- one membrane has a molecular weight cut-off of between about 300 to about 500 Dalton, and
- the other membrane as a molecular weight cut-off of between about 600 to about 800 Dalton.

In an alternative exemplary embodiment, the separation and purification of at least one of the produced neutral non-fucosylated oligosaccharides is made in a process, comprising the following steps in any order comprising the step of treating the cultivation or a clarified version thereof with a strong cation exchange resin in H+-form and a weak anion exchange resin in free base form.

In an alternative exemplary embodiment, the separation and purification of at least one of the produced neutral non-fucosylated oligosaccharides is made in the following way. The cultivation comprising the produced oligosaccharide, biomass, medium components and contaminants, and wherein the purity of the produced oligosaccharide in the fermentation broth is < 80 percent, is applied to the following purification steps:
i) separation of biomass from the cultivation,
ii) cationic ion exchanger treatment for the removal of positively charged material,
iii) anionic ion exchanger treatment for the removal of negatively charged material,
iv) nanofiltration step and/or electrodialysis step,
wherein a purified solution comprising the produced oligosaccharide(s) at a purity of greater than or equal to 80 percent is provided. Optionally the purified solution is spray dried.

In an alternative exemplary embodiment, the separation and purification of at least one of the produced neutral non-fucosylated oligosaccharides is made in a process, comprising the following steps in any order: enzymatic treatment of the cultivation; removal of the biomass from the cultivation; ultrafiltration; nanofiltration; and a column chromatography step. Preferably such column chromatography is a single column or a multiple column. Further preferably the column chromatography step is simulated moving bed chromatography. Such simulated moving bed chromatography preferably comprises i) at least 4 columns, wherein at least one column comprises a weak or strong cation exchange resin; and/or ii) four zones I, II, III and IV with different flow rates; and/or iii) an eluent comprising water; and/or iv) an operating temperature of 15 degrees to 60 degrees centigrade.

In a specific embodiment, the present invention provides the produced oligosaccharide or oligosaccharide mixture which is spray-dried to powder, wherein the spray-dried powder contains < 15 percent -wt. of water, preferably < 10 percent -wt. of water, more preferably < 7 percent -wt. of water, most preferably < 5 percent -wt. of water.

In a third aspect, the present invention provides for the use of a metabolically engineered cell as described herein for the production of a mixture comprising at least four different neutral non-fucosylated oligosaccharides. For identification of the oligosaccharides in the mixture comprising at least four neutral non-fucosylated different oligosaccharides produced in the cell as described herein, the monomeric building blocks (e.g. the monosaccharide or glycan unit composition), the anomeric configuration of side chains, the presence and location of substituent groups, degree of polymerization/molecular weight and the linkage pattern can be identified by standard methods known in the art, such as, e.g. methylation analysis, reductive cleavage, hydrolysis, GC-MS (gas chromatography-mass spectrometry), MALDI-MS (Matrix-assisted laser desorption/ionization-mass spectrometry), ESI-MS (Electrospray ionization-mass spectrometry), HPLC (High-Performance Liquid chromatography with ultraviolet or refractive index detection), HPAEC-PAD (High-Performance Anion-Exchange chromatography with Pulsed Amperometric Detection), CE (capillary electrophoresis), IR (infrared)/Raman spectroscopy, and NMR (Nuclear magnetic resonance) spectroscopy techniques. The crystal structure can be solved using, e.g., solid-state NMR, FT-IR (Fourier transform infrared spectroscopy), and WAXS (wide-angle X-ray scattering). The degree of polymerization (DP), the DP distribution, and polydispersity can be determined by, e.g., viscosimetry and SEC (SEC-HPLC, high performance size-exclusion chromatography). To identify the monomeric components of the saccharide methods such as, e.g. acid-catalyzed hydrolysis, HPLC (high performance liquid chromatography) or GLC (gas-liquid chromatography) (after conversion to alditol acetates) may be used. To determine the glycosidic linkages, the saccharide is methylated with methyl iodide and strong base in DMSO, hydrolysis is performed, a reduction to partially methylated alditols is achieved, an acetylation to methylated alditol acetates is performed, and the analysis is carried out by GLC/MS (gas-liquid chromatography coupled with mass spectrometry). To determine the oligosaccharide sequence, a partial depolymerization is carried out using an acid or enzymes to determine the structures. To identify the anomeric configuration, the oligosaccharide is subjected to enzymatic analysis, e.g. it is contacted with an enzyme that is specific for a particular type of linkage, e.g., beta-galactosidase, or alphaglucosidase, etc., and NMR may be used to analyze the products.

### Products comprising the oligosaccharide mixture

In some embodiments, an oligosaccharide mixture produced as described herein is incorporated into a food (e.g, human food or feed), dietary supplement, pharmaceutical ingredient, cosmetic ingredient or medicine. In some embodiments, the oligosaccharide mixture is mixed with one or more ingredients suitable for food, feed, dietary supplement, pharmaceutical ingredient, cosmetic ingredient or medicine. In some embodiments, the dietary supplement comprises at least one prebiotic ingredient and/or at least one probiotic ingredient.

A "prebiotic" is a substance that promotes growth of microorganisms beneficial to the host, particularly microorganisms in the gastrointestinal tract. In some embodiments, a dietary supplement provides multiple prebiotics, including the oligosaccharide mixture produced and/or purified by a process disclosed in this specification, to promote growth of one or more beneficial microorganisms. Examples of prebiotic ingredients for dietary supplements include other prebiotic molecules (such as HMOs) and plant polysaccharides (such as inulin, pectin, b- glucan and xylooligosaccharide). A "probiotic" product typically contains live microorganisms that replace or add to gastrointestinal microflora, to the benefit of the recipient. Examples of such microorganisms include Lactobacillus species (for example, L. acidophilus and L. bulgaricus), Bifidobacterium species (for example, B. animalis, B. longum and B. infantis (e.g., Bi-26)), and Saccharomyces boulardii. In some embodiments, an oligosaccharide mixture produced and/or purified by a process of this specification is orally administered in combination with such microorganism. Examples of further ingredients for dietary supplements include disaccharides (such as lactose), monosaccharides (such as glucose and galactose), thickeners (such as gum arabic), acidity regulators (such as trisodium citrate), water, skimmed milk, and flavourings.

In some embodiments, the oligosaccharide mixture is incorporated into a human baby food (e.g., infant formula). Infant formula is generally a manufactured food for feeding to infants as a complete or partial substitute for human breast milk. In some embodiments, infant formula is sold as a powder and prepared for bottle- or cup-feeding to an infant by mixing with water. The composition of infant formula is typically designed to be roughly mimic human breast milk. In some embodiments, an oligosaccharide mixture produced and/or purified by a process in this specification is included in infant formula to provide nutritional benefits similar to those provided by the oligosaccharides in human breast milk. In some embodiments, the oligosaccharide mixture is mixed with one or more ingredients of the infant formula. Examples of infant formula ingredients include nonfat milk, carbohydrate sources (e.g., lactose), protein sources (e.g., whey protein concentrate and casein), fat sources (e.g., vegetable oils - such as palm, high oleic safflower oil, rapeseed, coconut and/or sunflower oil; and fish oils), vitamins (such as vitamins A, Bb, Bi2, C and D), minerals (such as potassium citrate, calcium citrate, magnesium chloride, sodium chloride, sodium citrate and calcium phosphate) and possibly human milk oligosaccharides (HMOs). Such HMOs may include, for example, DiFL, lacto-N-triose II, LNT, LNnT, lacto-N-fucopentaose I, lacto-N-neofucopentaose, lacto-N-fucopentaose II, lacto-N- fucopentaose III, lacto-N-fucopentaose V, lacto-N-neofucopentaose V, lacto-N-difucohexaose I, lacto-N-difucohexaose II, 6'-galactosyllactose, 3'-galactosyllactose, lacto-N-hexaose and lacto- N-neohexaose.

In some embodiments, the one or more infant formula ingredients comprise nonfat milk, a carbohydrate source, a protein source, a fat source, and/or a vitamin and mineral.

In some embodiments, the one or more infant formula ingredients comprise lactose, whey protein concentrate and/or high oleic safflower oil.

In some embodiments, the oligosaccharide mixture's concentration in the infant formula is approximately the same concentration as the oligosaccharide's concentration generally present in human breast milk. In some embodiments, the concentration of each of the single oligosaccharides in the mixture of oligosaccharides in the infant formula is approximately the same concentration as the concentration of that oligosaccharide generally present in human breast milk.

In some embodiments, the oligosaccharide mixture is incorporated into a feed preparation, wherein said feed is chosen from the list comprising petfood, animal milk replacer, veterinary product, post weaning feed, or creep feed.

Unless defined otherwise, all technical and scientific terms used herein generally have the same meaning as commonly understood by one of ordinary skill in the art to which this invention belongs. Generally, the nomenclature used herein and the laboratory procedures in cell culture, molecular genetics, organic chemistry and nucleic acid chemistry and hybridization described above and below are those well-known and commonly employed in the art. Standard techniques are used for nucleic acid and peptide synthesis. Generally, purification steps are performed according to the manufacturer's specifications.

Further advantages follow from the specific embodiments and the examples. It goes without saying that the abovementioned features and the features which are still to be explained below can be used not only in the respectively specified combinations, but also in other combinations or on their own, without departing from the scope of the present invention.

The present invention relates to a metabolically engineered cell producing a mixture of at least four different neutral non-fucosylated oligosaccharides, wherein said cell
- expresses at least three glycosyltransferases, and
- is capable to synthesize one or more nucleotide-sugar(s), wherein said nucleotide-sugar(s) is/are donor(s) for said glycosyltransferases.

In an additional and/or alternative embodiment, said cell is modified with gene expression modules, characterized in that the expression from any of said expression modules is either constitutive or is created by a natural inducer. In an additional and/or alternative embodiment, said oligosaccharide mixture comprises at least three different neutral non-fucosylated oligosaccharides differing in degree of polymerization.

In an additional and/or preferred embodiment, any one of said glycosyltransferase is chosen from the list comprising galactosyltransferases, glucosyltransferases, mannosyltransferases, N-acetylglucosaminyltransferases, N-acetylgalactosaminyltransferases, N-acetylmannosaminyltransferases, xylosyltransferases, glucosaminyltransferases, rhamnosyltransferases. In an additional and/or alternative embodiment, said cell is modified in the expression or activity of at least one of said glycosyltransferases. In an additional and/or preferred embodiment, any one of said glycosyltransferases is an N-acetylglucosaminyltransferase and any one of said donor nucleotide-sugar is UDP-N-acetylglucosamine (UDP-GIcNAc). In an additional and/or preferred embodiment, any one of said glycosyltransferase is a galactosyltransferase and any one of said donor nucleotide-sugar is UDP-galactose (UDP-Gal). In an additional and/or preferred embodiment, any one of said nucleotide-sugar is chosen from the list comprising UDP-GIcNAc, UDP-Gal, UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, UDP-xylose. In an additional and/or preferred embodiment, said mixture comprises at least one neutral non-fucosylated oligosaccharide that is galactosylated, glucosylated, xylosylated, mannosylated, contains an N-acetylglucosamine, contains an N-acetylgalactosamine, contains a rhamnose, and/or contains an N-acetylmannosamine. In an additional and/or preferred embodiment, said oligosaccharide mixture comprises at least one oligosaccharide that comprises an N-acetylglucosamine monosaccharide unit. In an additional and/or preferred embodiment, said oligosaccharide mixture comprises at least one galactosylated oligosaccharide. In an additional and/or preferred embodiment, said cell is further genetically modified for
i) modified expression of an endogenous membrane protein, and/or
ii) modified activity of an endogenous membrane protein, and/or
iii) expression of a homologous membrane protein, and/or
iv) expression of a heterologous membrane protein,

wherein said membrane protein is involved in the secretion of any one of said neutral non-fucosylated oligosaccharides from said mixture outside said cell. In an additional and/or preferred embodiment, said cell is further genetically modified for
   i) modified expression of an endogenous membrane protein, and/or
   ii) modified activity of an endogenous membrane protein, and/or
   iii) expression of a homologous membrane protein, and/or
   iv) expression of a heterologous membrane protein,
wherein said membrane protein is involved in the uptake of a precursor for the synthesis of any one of said neutral non-fucosylated oligosaccharides. In an additional and/or preferred embodiment, said cell is producing a precursor for the synthesis of any one of said neutral non-fucosylated oligosaccharides. In an additional and/or preferred embodiment, any one of said neutral non-fucosylated oligosaccharide is a mammalian milk oligosaccharide. In an additional and/or preferred embodiment, all said neutral non-fucosylated oligosaccharides are mammalian milk oligosaccharides. In an additional and/or preferred embodiment, said cell is selected from the group consisting of microorganism, plant, or animal cells, preferably said microorganism is a bacterium, fungus or a yeast, preferably said plant is a rice, cotton, rapeseed, soy, maize or corn plant, preferably said animal is an insect, fish, bird or non-human mammal, preferably said animal cell is a mammalian cell line. In an additional and/or preferred embodiment, said cell is a cell of a bacterium, preferably of an *Escherichia coli* strain, more preferably of an *Escherichia coli* strain which is a K-12 strain, even more preferably the *Escherichia coli* K-12 strain is *E. coli* MG1655. In an additional and/or preferred embodiment, said cell is a yeast cell.

The present invention further relates to a method to produce a mixture of at least four different neutral non-fucosylated oligosaccharides by a cell, the method comprising the steps of:
i) providing a cell expressing at least three glycosyltransferases and capable to synthesize one or more nucleotide-sugar(s), wherein said nucleotide-sugar(s) is/are donor(s) for said glycosyltransferases,
ii) cultivating said cell under conditions permissive to express said glycosyltransferases and to synthesize said nucleotide-sugars,
iii) preferably, separating at least one of said neutral non-fucosylated oligosaccharides from said cultivation.

In an additional and/or preferred embodiment, said cell is a metabolically engineered cell as described herein. In an additional and/or preferred embodiment, said oligosaccharide mixture comprises at least three different neutral non-fucosylated oligosaccharides differing in degree of polymerization. In an additional and/or preferred embodiment, any one of said glycosyltransferases is chosen from the list comprising galactosyltransferases, glucosyltransferases, mannosyltransferases, N-acetylglucosaminyltransferases, N-acetylgalactosaminyltransferases, N-acetylmannosaminyltransferases, xylosyltransferases, glucosaminyltransferases, rhamnosyltransferases. In an additional and/or preferred embodiment, any one of said glycosyltransferases is an N-acetylglucosaminyltransferase and any one of said donor nucleotide-sugars is UDP-N-acetylglucosamine (UDP-GIcNAc). In an additional and/or preferred embodiment, any one of said glycosyltransferases is a galactosyltransferase and any one of said donor nucleotide-sugar is UDP-galactose (UDP-Gal). In an additional and/or preferred embodiment, any one of said nucleotide-sugars is chosen from the list comprising UDP-GIcNAc, UDP-Gal, UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, UDP-xylose. In an additional and/or preferred embodiment, said oligosaccharide mixture comprises at least one oligosaccharide that comprises an N-acetylglucosamine monosaccharide unit. In an additional and/or preferred embodiment, oligosaccharide mixture comprises at least one galactosylated oligosaccharide. In an additional and/or preferred embodiment, any one of said neutral non-fucosylated oligosaccharides is galactosylated, glucosylated, xylosylated, mannosylated, contains an N-acetylglucosamine, contains an N-acetylgalactosamine, contains a rhamnose, and/or contains an N-acetylmannosamine. In an additional and/or preferred embodiment, said cell uses at least one precursor for the synthesis of any one or more of said oligosaccharides, preferably said cell uses two or more precursors for the synthesis of any one or more of said oligosaccharides. In an additional and/or preferred embodiment, said cell is producing a precursor for the synthesis of any one of said neutral non-fucosylated oligosaccharides. In an additional and/or preferred embodiment, any one of said neutral non-fucosylated oligosaccharide is a mammalian milk oligosaccharide. In an additional and/or preferred embodiment, all said neutral non-fucosylated oligosaccharides are mammalian milk oligosaccharides. In an additional and/or preferred embodiment, said precursor for the synthesis of any one of said neutral non-fucosylated oligosaccharides is completely converted into any one of said neutral non-fucosylated oligosaccharides. In an additional and/or preferred embodiment, said separation comprises at least one of the following steps: clarification, ultrafiltration, nanofiltration, reverse osmosis, microfiltration, activated charcoal or carbon treatment, tangential flow high-performance filtration, tangential flow ultrafiltration, affinity chromatography, ion exchange chromatography, hydrophobic interaction chromatography and/or gel filtration, ligand exchange chromatography. In an additional and/or preferred embodiment, said method further comprising purification of any one of said neutral non-fucosylated oligosaccharides from said cell. In an additional and/or preferred embodiment, said purification comprises at least one of the following steps: use of activated charcoal or carbon, use of charcoal, nanofiltration, ultrafiltration or ion exchange, use of alcohols, use of aqueous alcohol mixtures, crystallization, evaporation, precipitation, drying, spray drying or lyophilization. In an additional and/or preferred embodiment, said cell is selected from the group consisting of microorganism, plant, or animal cells, preferably said microorganism is a bacterium, fungus or a yeast, preferably said plant is a rice, cotton, rapeseed, soy, maize or corn plant, preferably said animal is an insect, fish, bird or non-human mammal, preferably said animal cell is a mammalian cell line. In an additional and/or preferred embodiment, said cell is a cell of a bacterium, preferably of an *Escherichia coli* strain, more preferably of an *Escherichia coli* strain which is a K-12 strain, even more preferably the *Escherichia coli* K-12 strain is *E. coli* MG1655. In an additional and/or preferred embodiment, said cell is a yeast cell.

The present invention further relates to the use of a cell as described herein, or the use of a method as described herein, for the production of a mixture of at least four different neutral non-fucosylated oligosaccharides.

The invention will be described in more detail in the examples.

The following examples will serve as further illustration and clarification of the present invention and are not intended to be limiting.

### Examples

### Example 1. Materials and Methods Escherichia coli

### Media

The Luria Broth (LB) medium consisted of 1% tryptone peptone (Difco, Erembodegem, Belgium), 0.5% yeast extract (Difco) and 0.5% sodium chloride (VWR. Leuven, Belgium). The minimal medium used in the cultivation experiments in 96-well plates or in shake flasks contained 2.00 g/L NH4CI, 5.00 g/L (NH4)2SO4, 2.993 g/L KH2PO4, 7.315 g/L K2HPO4, 8.372 g/L MOPS, 0.5 g/L NaCl, 0.5 g/L MgSO4.7H2O, 30 g/L sucrose or 30 g/L glycerol, 1 ml/L vitamin solution, 100 µl/L molybdate solution, and 1 mL/L selenium solution. As specified in the respective examples, 20 g/L lactose, 20 g/L LacNAc and/or 20 g/L LNB were additionally added to the medium as precursor(s). The minimal medium was set to a pH of 7 with 1M KOH. Vitamin solution consisted of 3.6 g/L FeCl2.4H2O, 5 g/L CaCl2.2H2O, 1.3 g/L MnCl2.2H2O, 0.38 g/L CuCl2.2H2O, 0.5 g/L CoCl2.6H2O, 0.94 g/L ZnCl2, 0.0311 g/L H3BO4, 0.4 g/L Na2EDTA.2H2O and 1.01 g/L thiamine.HCl. The molybdate solution contained 0.967 g/L NaMoO4.2H2O. The selenium solution contained 42 g/L Seo2.

The minimal medium for fermentations contained 6.75 g/L NH4CI, 1.25 g/L (NH4)2SO4, 2.93 g/L KH2PO4 and 7.31 g/L KH2PO4, 0.5 g/L NaCl, 0.5 g/L MgSO4.7H2O, 30 g/L sucrose or 30 g/L glycerol, 1 mL/L vitamin solution, 100 µL/L molybdate solution, and 1 mL/L selenium solution with the same composition as described above. As specified in the respective examples, 20 g/L lactose, 20 g/L LacNAc and/or 20 g/L LNB were additionally added to the medium as precursor(s).

Complex medium was sterilized by autoclaving (121°C, 21 min) and minimal medium by filtration (0.22 µm Sartorius). When necessary, the medium was made selective by adding an antibiotic: e.g. chloramphenicol (20 mg/L), carbenicillin (100 mg/L), spectinomycin (40 mg/L) and/or kanamycin (50 mg/L).

### Plasmids

pKD46 (Red helper plasmid, Ampicillin resistance), pKD3 (contains an FRT-flanked chloramphenicol resistance (cat) gene), pKD4 (contains an FRT-flanked kanamycin resistance (kan) gene), and pCP20 (expresses FLP recombinase activity) plasmids were obtained from Prof. R. Cunin (Vrije Universiteit Brussel, Belgium in 2007). Plasmids were maintained in the host *E. coli* DH5alpha (F⁻, phi80d/*acZΔ*M15, Δ(*lacZYA-argF*) U169, *deoR, recA1, endA1,* hsdR17(rk⁻, mk⁺), *phoA, supE44,* lambda-, *thi*-1*, gyrA96, rel*A1) bought from Invitrogen.

### Strains and mutations

*Escherichia coli* K12 MG1655 [λ⁻, F⁻, rph-1] was obtained from the Coli Genetic Stock Center (US), CGSC Strain#: 7740, in March 2007. Gene disruptions, gene introductions and gene replacements were performed using the technique published by Datsenko and Wanner (PNAS 97 (2000), 6640-6645). This technique is based on antibiotic selection after homologous recombination performed by lambda Red recombinase. Subsequent catalysis of a flippase recombinase ensures removal of the antibiotic selection cassette in the final production strain. Transformants carrying a Red helper plasmid pKD46 were grown in 10 mL LB media with ampicillin, (100 mg/L) and L-arabinose (10 mM) at 30 °C to an OD₆₀₀nm of 0.6. The cells were made electrocompetent by washing them with 50 mL of ice-cold water, a first time, and with 1mL ice cold water, a second time. Then, the cells were resuspended in 50 µL of ice-cold water. Electroporation was done with 50 µL of cells and 10-100 ng of linear double-stranded-DNA product by using a Gene Pulser^{™} (BioRad) (600 Ω, 25 µFD, and 250 volts). After electroporation, cells were added to 1 mL LB media incubated 1 h at 37 °C, and finally spread onto LB-agar containing 25 mg/L of chloramphenicol or 50 mg/L of kanamycin to select antibiotic resistant transformants. The selected mutants were verified by PCR with primers upstream and downstream of the modified region and were grown in LB-agar at 42°C for the loss of the helper plasmid. The mutants were tested for ampicillin sensitivity. The linear ds-DNA amplicons were obtained by PCR using pKD3, pKD4 and their derivates as template. The primers used had a part of the sequence complementary to the template and another part complementary to the side on the chromosomal DNA where the recombination must take place. For the genomic knock-out, the region of homology was designed 50-nt upstream and 50-nt downstream of the start and stop codon of the gene of interest. For the genomic knock-in, the transcriptional starting point (+1) had to be respected. PCR products were PCR-purified, digested with Dpnl, re-purified from an agarose gel, and suspended in elution buffer (5 mM Tris, pH 8.0). Selected mutants were transformed with pCP20 plasmid, which is an ampicillin and chloramphenicol resistant plasmid that shows temperature- sensitive replication and thermal induction of FLP synthesis. The ampicillin-resistant transformants were selected at 30°C, after which a few were colony purified in LB at 42 °C and then tested for loss of all antibiotic resistance and of the FLP helper plasmid. The gene knock outs and knock ins are checked with control primers.

To produce lactose (Gal-b1,4-Glc), the mutant strain was derived from *E. coli* K12 MG1655 and modified with genomic knock-outs of the *E. coli lacZ, glk* genes and the *E. coli galETKM* operon, together with genomic knock-ins of constitutive transcriptional units for the N-acetylglucosamine beta-1,4-galactosyltransferase (IgtB) gene from *N. meningitidis* with SEQ ID NO 09 and the UDP-glucose 4-eprimerase (galE) from *E. coli* with SEQ ID NO 10 .

To produce lacto-N-triose (LN3, LNT-II, GIcNAc-b1,3-Gal-b1,4-Glc) and neutral non-fucosylated oligosaccharides originating thereof comprising lacto-*N*-tetraose (LNT) and lacto-*N*-neotetraose (LNnT), the mutant strain was derived from *E. coli* K12 MG1655 and modified with a knock-out of the *E. coli LacZ* and *nagB* genes and with a genomic knock-in of a constitutive transcriptional unit for the galactoside beta-1,3-N-acetylglucosaminyltransferase (LgtA) from *N. meningitidis* with SEQ ID NO 07. For LNT or LNnT production, the mutant strain is further modified with constitutive transcriptional units for the N-acetylglucosamine beta-1,3-galactosyltransferase (WbgO) from *E. coli* O55:H7 with SEQ ID NO 08 or the N-acetylglucosamine beta-1,4-galactosyltransferase (LgtB) from *N. meningitidis* with SEQ ID NO 09, respectively, that can be delivered to the strain either via genomic knock-in or from an expression plasmid. Optionally, multiple copies of the *LgtA, wbgO* and/or *LgtB* genes could be added to the mutant *E. coli* strains. Also, LNT and/or LNnT production can be enhanced by improved UDP-GIcNAc production by modification of the strains with one or more genomic knock-ins of a constitutive transcriptional unit for the mutant L-glutamine-D-fructose-6-phosphate aminotransferase glmS*54 from *E. coli* with SEQ ID NO 06 (differing from the wild-type glmS by an A39T, an R250C and an G472S mutation). In addition, the strains can optionally be modified for enhanced UDP-galactose production with genomic knock-outs of the *E. coli ushA* and *galT* genes. The mutant *E. coli* strains can also optionally be adapted with a genomic knock-in of a constitutive transcriptional unit for the UDP-glucose-4-epimerase (galE) from *E. coli* with SEQ ID NO 10, the phosphoglucosamine mutase (glmM) from *E. coli* with SEQ ID NO 17 and the N-acetylglucosamine-1-phosphate uridyltransferase / glucosamine-1-phosphate acetyltransferase (glmU) from *E. coli* with SEQ ID NO 18. The mutant strains could also optionally be adapted for growth on sucrose via genomic knock-ins of constitutive transcriptional units containing a sucrose transporter (CscB) from *E*. *coli* W with SEQ ID NO 01, a fructose kinase (Frk) originating from *Z*. *mobilis* with SEQ ID NO 02 and a sucrose phosphorylase originating from *B. adolescentis* with SEQ ID NO 03.

To produce N-acetyllactosamine (LacNAc, Gal-b-1,4-GIcNAc) or lacto-N-biose (LNB, Gal-b1,3-GlcNAc), the mutant strains were derived from *E. coli* K12 MG1655 comprising knock-outs of the *E. coli nagA* and *nagB* genes and a genomic knock-in of a constitutive transcriptional unit containing a glucosamine 6-phosphate N-acetyltransferase (GNA1) from *Saccharomyces cerevisiae* with SEQ ID NO 05. The mutant strains were further modified with constitutive transcriptional units for the N-acetylglucosamine beta-1,3-galactosyltransferase (WbgO) from *E. coli* O55:1-17 with SEQ ID NO 08 to produce LNB or the N-acetylglucosamine beta-1,4-galactosyltransferase (LgtB) from *N. meningitidis* with SEQ ID NO 09 to produce LacNAc. Those transcriptional units for WbgO and LgtB can be delivered to the strains either via genomic knock-in or from an expression plasmid. Optionally, multiple copies of the *wbgO* and/or *LgtB* genes could be added to the mutant *E. coli* strains. Optionally, the mutant strains can be modified with a constitutive transcriptional unit for the mutated variant of the L-glutamine-D-fructose-6-phosphate aminotransferase (glmS*54) from *E. coli* with SEQ ID NO 06 for enhanced UDP-GIcNAc synthesis. Optionally, the mutant strains can be further modified with a constitutive transcriptional unit for the phosphatase yqaB from *E. coli* with SEQ ID NO 19 for enhanced GlcNAc production. In addition, the strains can optionally be modified for enhanced UDP-galactose production with genomic knock-outs of the *E. coli ushA* and *galT* genes. The mutant *E. coli* strains can also optionally be adapted with a genomic knock-in of a constitutive transcriptional unit for the UDP-glucose-4-epimerase (galE) from *E. coli* with SEQ ID NO 10. The mutant strains can also optionally be adapted for growth on sucrose via genomic knock-ins of constitutive transcriptional units containing a sucrose transporter (CscB) from *E. coli* W with SEQ ID NO 01, a fructose kinase (Frk) originating from *Z*. *mobilis* with SEQ ID NO 02 and a sucrose phosphorylase originating from *B*. *adolescentis* with SEQ ID NO 03.

Preferably but not necessarily, the glycosyltransferases were N-terminally fused to an MBP-tag to enhance their solubility (Fox et al., Protein Sci. 2001, 10(3), 622-630).

All constitutive promoters, UTRs and terminator sequences originated from the libraries described by Mutalik et al. (Nat. Methods 2013, No. 10, 354-360) and Cambray et al. (Nucleic Acids Res. 2013, 41(9), 5139-5148). All genes were ordered synthetically at Twist Bioscience (twistbioscience.com) or IDT (eu.idtdna.com) and the codon usage was adapted using the tools of the supplier.

All strains were stored in cryovials at -80°C (overnight LB culture mixed in a 1:1 ratio with 70% glycerol).

### Cultivation conditions

A preculture of 96-well microtiter plate experiments was started from a cryovial, in 150 µL LB and was incubated overnight at 37 °C on an orbital shaker at 800 rpm. This culture was used as inoculum for a 96well square microtiter plate, with 400 µL minimal medium by diluting 400x. These final 96-well culture plates were then incubated at 37°C on an orbital shaker at 800 rpm for 72h, or shorter, or longer. To measure sugar concentrations at the end of the cultivation experiment whole broth samples were taken from each well by boiling the culture broth for 15 min at 60°C before spinning down the cells (= average of intra- and extracellular sugar concentrations).

A preculture for the bioreactor was started from an entire 1 mL cryovial of a certain strain, inoculated in 250 mL or 500 mL minimal medium in a 1 Lor 2.5 L shake flask and incubated for 24 h at 37°C on an orbital shaker at 200 rpm. A 5 L bioreactor was then inoculated (250 mL inoculum in 2 L batch medium); the process was controlled by MFCS control software (Sartorius Stedim Biotech, Melsungen, Germany). Culturing condition were set to 37 °C, and maximal stirring; pressure gas flow rates were dependent on the strain and bioreactor. The pH was controlled at 6.8 using 0.5 M H2S04 and 20% NH4OH. The exhaust gas was cooled. 10% solution of silicone antifoaming agent was added when foaming raised during the fermentation.

### Optical density

Cell density of the cultures was frequently monitored by measuring optical density at 600 nm (Implen Nanophotometer NP80, Westburg, Belgium or with a Spark 10M microplate reader, Tecan, Switzerland).

### Analytical analysis

Standards such as but not limited to sucrose, lactose, N-acetyllactosamine (LacNAc), lacto-N-biose (LNB), lacto-*N*-triose II (LN3), lacto-N-tetraose (LNT) and lacto-*N*-neo-tetraose (LNnT) were purchased from Carbosynth (UK), Elicityl (France) and IsoSep (Sweden). Other compounds were analyzed with in-house made standards.

Oligosaccharides were analyzed on a Waters Acquity H-class UPLC with Evaporative Light Scattering Detector (ELSD) or a Refractive Index (RI) detection. A volume of 0.7 µL sample was injected on a Waters Acquity UPLC BEH Amide column (2.1 × 100 mm;130 Å;1.7 µm) column with an Acquity UPLC BEH Amide VanGuard column, 130 Å, 2.1× 5 mm. The column temperature was 50°C. The mobile phase consisted of a % water and % acetonitrile solution to which 0.2 % triethylamine was added. The method was isocratic with a flow of 0.130 mL/min. The ELS detector had a drift tube temperature of 50 °C and the N2 gas pressure was 50 psi, the gain 200 and the data rate 10 pps. The temperature of the RI detector was set at 35 °C.

Oligosaccharides were also analyzed on a Waters Acquity H-class UPLC with Refractive Index (RI) detection. A volume of 0.5 µL sample was injected on a Waters Acquity UPLC BEH Amide column (2.1 × 100 mm;130 Å;1.7 µm). The column temperature was 50°C. The mobile phase consisted of a mixture of 72% acetonitrile and 28% ammonium acetate buffer (100 mM) to which 0.1% triethylamine was added. The method was isocratic with a flow of 0.260 mL/min. The temperature of the RI detector was set at 35 °C.

For analysis on a mass spectrometer, a Waters Xevo TQ-MS with Electron Spray lonisation (ESI) was used with a desolvation temperature of 450 °C, a nitrogen desolvation gas flow of 650 L/h and a cone voltage of 20 V. The MS was operated in selected ion monitoring (SIM) in negative mode for all oligosaccharides. Separation was performed on a Waters Acquity UPLC with a Thermo Hypercarb column (2.1 × 100 mm; 3 µm) on 35 °C. A gradient was used wherein eluent A was ultrapure water with 0.1 % formic acid and wherein eluent B was acetonitrile with 0.1 % formic acid. The oligosaccharides were separated in 55 min using the following gradient: an initial increase from 2 to 12 % of eluent B over 21 min, a second increase from 12 to 40 % of eluent B over 11 min and a third increase from 40 to 100 % of eluent B over 5 min. As a washing step 100 % of eluent B was used for 5 min. For column equilibration, the initial condition of 2 % of eluent B was restored in 1 min and maintained for 12 min.

Sugars at low concentrations (below 50 mg/L) were analyzed on a Dionex HPAEC system with pulsed amperometric detection (PAD). A volume of 5 µL of sample was injected on a Dionex CarboPac PA200 column 4 x 250 mm with a Dionex CarboPac PA200 guard column 4 x 50 mm. The column temperature was set to 30 °C. A gradient was used wherein eluent A was deionized water, wherein eluent B was 200 mM Sodium hydroxide and wherein eluent C was 500 mM Sodium acetate. The oligosaccharides were separated in 60 min while maintaining a constant ratio of 25 % of eluent B using the following gradient: an initial isocratic step maintained for 10 min of 75 % of eluent A, an initial increase from 0 to 4 % of eluent C over 8 min, a second isocratic step maintained for 6 min of 71 % of eluent A and 4 % of eluent C, a second increase from 4 to 12 % of eluent Cover 2.6 min, a third isocratic step maintained for 3.4 min of 63 % of eluent A and 12 % of eluent C and a third increase from 12 to 48 % of eluent Cover 5 min. As a washing step 48 % of eluent C was used for 3 min. For column equilibration, the initial condition of 75 % of eluent A and 0 % of eluent C was restored in 1 min and maintained for 11 min. The applied flow was 0.5 mL/min.

### Example 2. Materials and Methods Saccharomyces cerevisiae

### Media

Strains were grown on Synthetic Defined yeast medium with Complete Supplement Mixture (SD CSM) or CSM drop-out (SD CSM-His) containing 6.7 g/L Yeast Nitrogen Base without amino acids (YNB w/o AA, Difco), 20 g/L agar (Difco) (solid cultures), 22 g/L glucose monohydrate or 20 g/L lactose and 0.79 g/L CSM or 0.77 g/L CSM-His (MP Biomedicals).

### Strains

*S. cerevisiae* BY4742 created by Brachmann et al. (Yeast (1998) 14:115-32) was used, available in the Euroscarf culture collection. All mutant strains were created by homologous recombination or plasmid transformation using the method of Gietz (Yeast 11:355-360, 1995).

### Plasmids

To produce UDP-galactose, a yeast expression plasmid was derived from the pRS420-plasmid series (Christianson et al., 1992, Gene 110: 119-122) containing the HIS3 selection marker and a constitutive transcriptional unit for galE from *E. coli* with SEQ ID NO 10. To produce LN3 and LN3-derived oligosaccharides like LNT or LNnT, this plasmid was further modified with constitutive transcriptional units for the lactose permease LAC12 from *K. lactis* with SEQ ID NO 11, the galactoside beta-1,3-N-acetylglucosaminyltransferase (IgtA) from *N. meningitidis* with SEQ ID NO 07 and, the N-acetylglucosamine beta-1,3-galactosyltransferase (WbgO) from *E. coli* O55:H7 with SEQ ID NO 08 or the N-acetylglucosamine beta-1,4-galactosyltransferase (IgtB) from *N. meningitidis* with SEQ ID NO 09, respectively.

Preferably but not necessarily, the glycosyltransferases were N-terminally fused to a SUMOstar tag (e.g. obtained from pYSUMOstar, Life Sensors, Malvern, PA) to enhance their solubility.

Plasmids were maintained in the host *E. coli* DH5alpha (F⁻, phi80d*lacZ*deltaM15, delta(*lacZYA-argF*)U169, *deoR, recA1, endA1,* hsdR17(rk⁻, mk⁺), *phoA, supE44,* lambda⁻, *thi*-1, *gyrA96, rel*A1) bought from Invitrogen.

### Heterologous and homologous expression

Genes that needed to be expressed, be it from a plasmid or from the genome were synthetically synthetized with one of the following companies: DNA2.0, Gen9, IDT or Twist Bioscience. Expression could be further facilitated by optimizing the codon usage to the codon usage of the expression host. Genes were optimized using the tools of the supplier.

### Cultivations conditions

In general, yeast strains were initially grown on SD CSM plates to obtain single colonies. These plates were grown for 2-3 days at 30 °C. Starting from a single colony, a preculture was grown over night in 5 mL at 30 °C, shaking at 200 rpm. Subsequent 125 mL shake flask experiments were inoculated with 2% of this preculture, in 25 mL media. These shake flasks were incubated at 30 °C with an orbital shaking of 200 rpm.

### Gene expression promoters

Genes were expressed using synthetic constitutive promoters, as described by Blazeck (Biotechnology and Bioengineering, Vol. 109, No. 11, 2012).

### Example 3 (reference example). Production of an oligosaccharide mixture comprising GIcNAc-b1,3-Gal-b1,4-GIcNAc and poly-LacNAc (Gal-b1,4-GlcNAc)n structures with a modified E. coli host

An *E. coli* K-12 MG1655 strain was modified with a knock-out of the *E. coli nagB* gene together with genomic knock-ins of constitutive transcriptional units for the phosphatase yqaB from *E. coli* with SEQ ID NO 19, GNA1 with SEQ ID NO 05 from S. *cerevisiae* and LgtB with SEQ ID NO 09 from *N. meningitidis* to produce LacNAc. In a next step, the novel strain was additionally transformed with an expression plasmid containing a constitutive transcriptional unit for the galactoside beta-1,3-N-acetylglucosaminyltransferase (LgtA) from *N. meningitidis* with SEQ ID NO 07. By subsequent action of the homologous EcGImS, EcGImM and EcGImU enzymes, the mutant strain produces UDP-GIcNAc, which is used by the heterologous LgtA protein to modify LacNAc leading to production of GIcNAc-b1,3-Gal-b1,4-GIcNAc and poly-LacNAc structures (Gal-b1,4-GlcNAc)n which are built of repeated N-acetyllactosamine units that are beta1,3-linked to each other, in whole broth samples when evaluated in a growth experiment according to the culture conditions provided in Example 1, in which the culture medium contains glycerol as carbon source.

### Example 4. Production of an oligosaccharide mixture comprising GIcNAc-b1,3-Gal-b1,4-GIcNAc, beta-Gal-(1,4)-beta-GlcNAc-(1,3)-[beta-GlcNAc-(1,6)]-beta-Gal-(1,4)-GlcNAc and poly-LacNAc structures with a modified E. coli host

The mutant *E. coli* strain as described in Example 3 was further modified with a second expression plasmid containing a constitutive transcriptional unit for the human N-acetyllactosaminide beta-1,6-N-acetylglucosaminyltransferase GCNT2 with SEQ ID NO 12. When evaluated in a growth experiment according to the culture conditions provided in Example 1 in which the culture medium contains glycerol as carbon source, the novel strain produces an oligosaccharide mixture comprising LacNAc, GIcNAc-b1,3-Gal-b1,4-GlcNAc, GlcNAc-b1,6-Gal-b1,4-GlcNAc, (Gal-b1,4-GlcNAc)n structures and beta-Gal-(1,4)-beta-GIcNAc-(1,3)-[beta-GIcNAc-(1,6)]-beta-Gal-(1,4)-GIcNAc in whole broth samples.

### Example 5. Production of an oligosaccharide mixture comprising Gal-a1,3-LacNAc, LN3, LNnT and Gala1,3-LNnT with a modified E. coli host

An *E. coli* strain modified to produce LacNAc as described in Example 3 was further modified with a genomic knock-out of the *E. coli lacZ* gene and transformed with a compatible expression plasmid containing a constitutive transcriptional unit for the C-terminal catalytic domain of the bovine alpha-1,3-galactosyltransferase (a3FTcd) with SEQ ID NO 13. The novel strain produces an oligosaccharide mixture comprising Gal-a1,3-LacNAc (Gal-a1,3-Gal-b1,4-GIcNAc), LN3, LNnT and Gal-a1,3-LNnT (Gal-a1,3-Gal-b1,4-GlcNAc-b1,3-Gal-b1,4-Glc) in whole broth samples when evaluated in a growth experiment according to the culture conditions provided in Example 1, in which the culture medium contains glycerol as carbon source and lactose as precursor.

### Example 6. Production of an oligosaccharide mixture comprising LacNAc, GaINAc-b1,3-lactose, Gal-b1,3-GaINAc-b1,3-lactose, LN3, LNnT, GaINAc-b1,3-LacNAc, Gal-b1,3-GaINAc-b1,3-LacNAc, poly-LacNAc (Galb1,4-GIcNAc)n and GalNAc-ylated poly-LacNAc structures with a modified E. coli host

An *E. coli* K-12 MG1655 strain was modified with a knock-out of the *E. coli nagB* and *lacZ* genes together with genomic knock-ins of constitutive transcriptional units for the mutant glmS^{∗}54 with SEQ ID NO 06 from *E. coli,* GNA1 with SEQ ID NO 05 from S. *cerevisiae,* the phosphatase yqaB from *E. coli* with SEQ ID NO 19, the galactoside beta-1,3-N-acetylglucosaminyltransferase (LgtA) from *N. meningitidis* with SEQ ID NO 07, LgtB with SEQ ID NO 09, the 4-epimerase (WbpP) of *Pseudomonas aeruginosa* with SEQ ID NO 14 and the β1,3-N-acetylgalactosaminyltransferase (LgtD) from *Haemophilus influenzae* with SEQ ID NO 15. When evaluated in a growth experiment according to the culture conditions provided in Example 1, in which the culture medium comprises glycerol as carbon source and lactose as precursor, the novel strain produces an oligosaccharide mixture comprising LacNAc, LN3, LNnT, GaINAc-b1,3-lactose, Gal-b1,3-GalNAc-b1,3-lactose, GalNAc-b1,3-Gal-b1,4-GlcNAc-b1,3-Gal-b1,4-Glc, GalNAc-b1,3-LacNAc, Gal-b1,3-GaINAc-b1,3-LacNAc, poly-LacNAc structures, i.e. (Gal-b1,4-GlcNAc)n and GalNAc-ylated poly-LacNAc structures.

### Example 7. Production of an oligosaccharide mixture comprising LNB, LN3, LNT, GaINAc-b1,3-lactose, Galb1,3- GalNAc-b1,3-lactose, GaINAc-b1,3-LNB and Gal-b1,3-GaINAc-b1,3-LNB with a modified E. coli host

An *E. coli* strain was modified with genomic knock-out of the *E. coli nagB* gene and genomic knock-ins of constitutive expression cassettes for the phosphatase yqaB from *E. coli* with SEQ ID NO 19, GNA1 with SEQ ID NO 05 from S. *cerevisiae* and WbgO with SEQ ID NO 08 from *E. coli* O55:H7 to produce LNB. In a next step, the LNB producing *E. coli* strain is further modified with a knock-out of the *E. coli lacZ* gene and with knock-ins of constitutive expression units for the 4-epimerase (WbpP) of *P. aeruginosa* with SEQ ID NO 14, the galactoside beta-1,3-N-acetylglucosaminyltransferase (LgtA) from *N. meningitidis* with SEQ ID NO 07 and the β1,3-N-acetylgalactosaminyltransferase (LgtD) from *H. influenzae* with SEQ ID NO 15. When evaluated in a growth experiment according to the culture conditions provided in Example 1, in which the culture medium comprises glycerol as carbon source and lactose as precursor, the novel strain produces an oligosaccharide mixture comprising LNB, LN3, LNT, GalNAc-b1,3-lactose, Gal-b1,3-GaINAc-b1,3-lactose, GalNAc-b1,3-Gal-b1,3-GlcNAc-b1,3-Gal-b1,4-Glc, GaINAc-b1,3-LNB and Gal-b1,3-GalNAc-b1,3-LNB.

### Example 8. Production of an oligosaccharide mixture comprising LNT, LNnT and poly-galactosylated structures in a modified E. coli host when evaluated in fed-batch fermentations

An *E. coli* strain modified to produce LNnT as described in Example 1, is further modified with genomic knock-ins of constitutive transcriptional units for WbgO from *E. coli* O55:H7 with SEQ ID NO 08. In a next step, the novel strain is evaluated in a fed-batch fermentation process in a 5L bioreactor as described in Example 1. In this example sucrose is used as a carbon source and lactose is added in the batch medium as precursor. Regular broth samples are taken, and sugars produced are measured as described in Example 1. UPLC analysis shows that fermentation broth of the selected strain taken at regular timepoints in fed-batch phase contains an oligosaccharide mixture comprising Lacto-N-triose II (LN3), Lacto-N-neotetraose (LNnT), Lacto-N-tetraose (LNT), para-Lacto-N-neopentaose, para-Lacto-N-pentaose, para-Lacto-N-neohexaose, para-Lacto-N-hexaose, beta-(1,3)Galactosyl-para-Lacto-N-neopentaose and beta-(1,4)Galactosyl-para-Lacto-N-pentaose.

### Example 9. Production of an oligosaccharide mixture comprising galactosylated and GalNAc-ylated lactose structures with a modified E. coli host

An *E. coli* strain optimized for UDP-galactose as described in Example 1, is further modified with a knock-out of the *E. coli lacZ* gene and a knock-in of a constitutive expression unit for the a1,4-galactosyltransferase (LgtC) from *Neisseria gonorrhoeae* with SEQ ID NO 16 to produce alpha-1,4-galactosylated lactose (Gal-a1,4-Gal-b1,4-Glc) when grown on glycerol and lactose. In a next step, the mutant strain is transformed with an expression plasmid comprising constitutive expression units for WbpP of *P. aeruginosa* with SEQ ID NO 14 and LgtD from *H. influenzae* with SEQ ID NO 15. Besides its β1,3-N-Acetyl-galactosaminyltransferase activity the HiLgtD enzyme also has a β1,3-galactosetransferase activity and can add a galactose to a non-reducing terminal GaINAc molecule resulting in a terminal Galb1,3-GalNAc at the non-reducing end of a glycan. The novel strain produces an oligosaccharide mixture comprising Gal-a1,4-Gal-b1,4-Glc (Gal-a1,4-lactose), GaINAc-b1,3-Gal-b1,4-Glc (GaINAc-b1,3-Lactose), Gal-b1,3-GaINAc-b1,3-lactose, GaINAc-b1,3-Gal-a1,4-Gal-b1,4-Glc (globo-N-tetraose) and Gal-b1,3-GaINAc-b1,3-Gal-a1,4-Gal-b1,4-Glc when evaluated in a growth experiment according to the culture conditions provided in Example 1, in which the culture medium contains glycerol as carbon source and lactose as precursor.

### Example 10. Production of an oligosaccharide mixture comprising LN3, LNT, GaINAc-b1,3-LNT, Gal-b1,3-GaINAc-b1,3-LNT, GaINAc-b1,3-lactose and Gal-b1,3-GaINAc-b1,3-lactose with a modified E. coli host

An *E. coli* strain modified to produce LNT as described in Example 1, is further modified with knock-ins of constitutive expression units for WbpP from *P. aeruginosa* with SEQ ID NO 14 and LgtD from *H. influenzae* with SEQ ID NO 15. The novel strain produces an oligosaccharide mixture comprising LN3, LNT, GaINAc-b1,3-LNT, Gal-b1,3-GaINAc-b1,3-LNT, GaINAc-b1,3-lactose and Gal-b1,3-GaINAc-b1,3-lactose in whole broth samples when evaluated in a growth experiment, according to the culture conditions in Example 1, in which the culture medium contains glycerol as carbon source and lactose as precursor.

### Example 11. Production an oligosaccharide mixture comprising LN3, LNnT, GaINAc-b1,3-LNnT, Gal-b1,3-GaINAc-b1,3-LNnT, GaINAc-b1,3-lactose, Gal-b1,3-GaINAc-b1,3-lactose and (GaINAc)-poly-LNnT structures with a modified E. coli host

An *E. coli* strain modified to produce LNnT as described in Example 1, is further modified with knock-ins of constitutive expression units for WbpP from *P. aeruginosa* with SEQ ID NO 14 and LgtD from *H*. *influenzae* with SEQ ID NO 15. The novel strain produces an oligosaccharide mixture comprising LN3, LNnT, GalNAc-b1,3-LNnT, Gal-b1,3-Gal NAc-b1,3-LN nT, GalNAc-b1,3-lactose and Gal-b1,3-GalNAc-b1,3-lactose as well as poly-LNnT structures (by alternate activity of NmlgtA and NmLgtB) and GalNAc-ylated poly-LNnT structures (by additional activity of HiLgtD) in whole broth samples when evaluated in a growth experiment, according to the culture conditions in Example 1, in which the culture medium contains glycerol as carbon source and lactose as precursor.

### Example 12. Production of an oligosaccharide mixture comprising Gal-a1,3-LacNAc, LN3, LNnT and Gala1,3-LNnT with a modified E. coli host

An E. coli K-12 MG1655 strain was modified with genomic knock-outs of the *E. coli lacZ, glk* genes and the *E. coli gaIETKM* operon, together with genomic knock-ins of constitutive transcriptional units for IgtB from *N. meningitidis* with SEQ ID NO 09 and the UDP-glucose 4-eprimerase (galE) from *E. coli* with SEQ ID NO 10 to produce lactose (Gal-b1,4-Glc). In next steps, the mutant strain was further modified with a knock-out of the *E. coli nagB* gene together with genomic knock-ins of constitutive transcriptional units for the mutant glmS^{∗}54 with SEQ ID NO 06 from *E. coli* and GNA1 with SEQ ID NO 05 from S. *cerevisiae* to produce GIcNAc and LacNAc. In a final step, the novel strain was additionally transformed with an expression plasmid containing constitutive transcriptional units for LgtA from *N. meningitidis* with SEQ ID NO 07 and the C-terminal catalytic domain of the bovine alpha-1,3-galactosyltransferase (a3FTcd) with SEQ ID NO 13. The novel strain produces an oligosaccharide mixture comprising Gal-a1,3-lactose, Gal-a1,3-LacNAc (Gal-a1,3-Gal-b1,4-GIcNAc), LN3, LNnT and Gal-a1,3-LNnT (Gal-a1,3-Gal-b1,4-GlcNAc-b1,3-Gal-b1,4-Glc) in whole broth samples when evaluated in a growth experiment according to the culture conditions provided in Example 1, in which the culture medium contains glycerol as carbon source.

### Example 13. Production of an oligosaccharide mixture comprising LacNAc, GaINAc-b1,3-lactose (b3'-GaINAcL), Gal-b1,3-GaINAc-b1,3-lactose, LN3, LNnT, GaINAc-b1,3-LacNAc, Gal-b1,3-GaINAc-b1,3-LacNAc, poly-LacNAc (Gal-b1,4-GIcNAc)n and GalNAc-ylated poly-LacNAc structures with a modified E. coli host

An E. coli K-12 MG1655 strain was modified with genomic knock-outs of the *E. coli lacZ, glk* genes and the *E. coli gaIETKM* operon, together with genomic knock-ins of constitutive transcriptional units for IgtB from *N. meningitidis* with SEQ ID NO 09 and the UDP-glucose 4-eprimerase (galE) from *E. coli* with SEQ ID NO 10 to produce lactose (Gal-b1,4-Glc). In next steps, the mutant strain was further modified with a knock-out of the *E. coli nagB* gene together with genomic knock-ins of constitutive transcriptional units for the mutant glmS*54 with SEQ ID NO 06 from *E. coli* and GNA1 with SEQ ID NO 05 from S. *cerevisiae* to produce GIcNAc and LacNAc. In a final step, the novel strain was additionally transformed with an expression plasmid containing constitutive transcriptional units for WbpP of *P. aeruginosa* with SEQ ID NO 14, LgtA from *N. meningitidis* with SEQ ID NO 07 and LgtD from *H. influenzae* with SEQ ID NO 15. When evaluated in a growth experiment according to the culture conditions provided in Example 1, in which the culture medium comprises glycerol as carbon source, the novel strain produces an oligosaccharide mixture comprising LacNAc, LN3, LNnT, GaINAc-b1,3-lactose, Gal-b1,3-GaINAc-b1,3-lactose, GaINAc-b1,3-Gal-b1,4-GlcNAc-b1,3-Gal-b1,4-Glc, GalNAc-b1,3-LacNAc, Gal-b1,3-GalNAc-b1,3-LacNAc, poly-LacNAc structures, i.e. (Gal-b1,4-GlcNAc)n and GalNAc-ylated poly-LacNAc structures.

### Example 14. Production of an oligosaccharide mixture comprising galactosylated and GalNAc-ylated lactose structures with a modified E. coli host

An E. coli K-12 MG1655 strain was modified with genomic knock-outs of the *E. coli nagB, ushA, galT, lacZ, glk* genes and the *E. coli gaIETKM* operon, together with genomic knock-ins of constitutive transcriptional units for IgtB from *N. meningitidis* with SEQ ID NO 09, galE from *E. coli* with SEQ ID NO 10, the a1,4-galactosyltransferase (LgtC) from *N. gonorrhoeae* with SEQ ID NO 16 and the mutant glmS*54 of *E. coli* with SEQ ID NO 06. In a next step, the mutant strain was transformed with an expression plasmid comprising constitutive expression units for WbpP of *P. aeruginosa* with SEQ ID NO 14 and LgtD from *H*. *influenzae* with SEQ ID NO 15. The novel strain produces an oligosaccharide mixture comprising Gal-a1,4-Gal-b1,4-Glc (Gal-a1,4-lactose), Gal-a1,4-Gal-a1,4-Gal-b1,4-Glc, Gal-a1,4-Gal-a1,4-Gal-a1,4-Gal-b1,4-Glc, GalNAc-b1,3-Gal-b1,4-Glc (GalNAc-b1,3-Lactose), Gal-b1,3-GalNAc-b1,3-lactose, GalNAc-b1,3-Gal-a1,4-Gal-b1,4-Glc (globo-N-tetraose) and Gal-b1,3-GaINAc-b1,3-Gal-a1,4-Gal-b1,4-Glc when evaluated in a growth experiment according to the culture conditions provided in Example 1, in which the culture medium contains glycerol as carbon source.

### Example 15. Production an oligosaccharide mixture comprising LN3, LNnT, GaINAc-b1,3-LNnT, Gal-b1,3-GaINAc-b1,3-LNnT, GaINAc-b1,3-lactose, Gal-b1,3-GaINAc-b1,3-lactose and (GaINAc)-poly-LNnT structures with a modified E. coli host

An E. coli K-12 MG1655 strain was modified with genomic knock-outs of the *E. coli nagB, lacZ, glk* genes and the *E. coli gaIETKM* operon, together with genomic knock-ins of constitutive transcriptional units for IgtB from *N. meningitidis* with SEQ ID NO 09, galE from *E. coli* with SEQ ID NO 10, IgtA from *N. meningitidis* with SEQ ID NO 07, the mutant glmS*54 of *E. coli* with SEQ ID NO 06, WbpP from *P. aeruginosa* with SEQ ID NO 14 and LgtD from *H. influenzae* with SEQ ID NO 15. The novel strain produces an oligosaccharide mixture comprising LN3, LNnT, GaINAc-b1,3-LNnT, Gal-b1,3-GaINAc-b1,3-LNnT, GaINAc-b1,3-lactose and Gal-b1,3-GaINAc-b1,3-lactose as well as poly-LNnT structures and GalNAc-ylated poly-LNnT structures in whole broth samples when evaluated in a growth experiment, according to the culture conditions in Example 1, in which the culture medium contains glycerol as carbon source.

### Example 16 reference example). Production of an oligosaccharide mixture comprising GIcNAc-b1,3-Gal-b1,4-GIcNAc and poly-LacNAc (Gal-b1,4-GlcNAc)n structures with a modified S. cerevisiae host

An S. *cerevisiae* strain was transformed with a yeast expression pRS420-plasmid containing the HIS3 selection marker and comprising constitutive transcriptional units for the mutant glmS*54 with SEQ ID NO 06 from *E. coli,* the phosphatase yqaB from *E. coli* with SEQ ID NO 19, galE from *E. coli* with SEQ ID NO 10, LgtA from *N. meningitidis* with SEQ ID NO 07 and LgtB with SEQ ID NO 09 from *N. meningitidis.* When evaluated on SD CSM-Ura-His drop-out medium, the mutant yeast strain produces an oligosaccharide mixture comprising LacNAc, GlcNAc-b1,3-Gal-b1,4-GlcNAc and poly-LacNAc structures (Gal-b1,4-GlcNAc)n.

### Example 17. Production of an oligosaccharide mixture comprising GIcNAc-b1,3-Gal-b1,4-GIcNAc, beta-Gal-(1,4)-beta-GlcNAc-(1,3)-[beta-GlcNAc-(1,6)]-beta-Gal-(1,4)-GlcNAc and poly-LacNAc structures with a modified S. cerevisiae host

An S. *cerevisiae* strain was transformed with a yeast expression pRS420-plasmid containing the HIS3 selection marker and comprising constitutive transcriptional units for the mutant glmS*54 with SEQ ID NO 06 from *E. coli,* the phosphatase yqaB from *E. coli* with SEQ ID NO 19, galE from *E. coli* with SEQ ID NO 10, LgtA from *N. meningitidis* with SEQ ID NO 07, LgtB with SEQ ID NO 09 from *N. meningitidis* and the human N-acetyllactosaminide beta-1,6-N-acetylglucosaminyltransferase GCNT2 with SEQ ID NO 12. When evaluated on SD CSM-Ura-His drop-out medium, the mutant yeast strain produces an oligosaccharide mixture comprising LacNAc, GIcNAc-b1,3-Gal-b1,4-GIcNAc, GlcNAc-b1,6-Gal-b1,4-GlcNAc, (Gal-b1,4-GlcNAc)n structures and beta-Gal-(1,4)-beta-GIcNAc-(1,3)-[beta-GIcNAc-(1,6)]-beta-Gal-(1,4)-GIcNAc.

### Example 18. Production of an oligosaccharide mixture comprising Gal-a1,3-LacNAc, LN3, LNnT and Gala1,3-LNnT with a modified S. cerevisiae host

An S. *cerevisiae* strain was transformed with a yeast expression pRS420-plasmid containing the HIS3 selection marker and comprising constitutive transcriptional units for the mutant glmS*54 with SEQ ID NO 06 from *E. coli,* the phosphatase yqaB from *E. coli* with SEQ ID NO 19, galE from *E. coli* with SEQ ID NO 10, LgtA from *N. meningitidis* with SEQ ID NO 07, LgtB with SEQ ID NO 09 from *N. meningitidis* and the C-terminal catalytic domain of the bovine alpha-1,3-galactosyltransferase (a3FTcd) with SEQ ID NO 13. When evaluated on SD CSM-Ura-His drop-out medium, the mutant yeast strain produces an oligosaccharide mixture comprising Gal-a1,3-LacNAc (Gal-a1,3-Gal-b1,4-GIcNAc), LN3, LNnT and Gal-a1,3-LNnT (Gal-a1,3-Gal-b1,4-GIcNAc-b1,3-Gal-b1,4-Glc).

### Example 19. Production of an oligosaccharide mixture comprising LacNAc, GaINAc-b1,3-lactose, Gal-b1,3-GaINAc-b1,3-lactose, LN3, LNnT, GaINAc-b1,3-LacNAc, Gal-b1,3-GaINAc-b1,3-LacNAc, poly-LacNAc (Gal-b1,4-GIcNAc)n and GaINAc-ylated poly-LocNAc structures with a modified S. cerevisiae host

An S. *cerevisiae* strain was transformed with a yeast artificial chromosome (YAC) comprising constitutive transcriptional units for the mutant glmS*54 with SEQ ID NO 06 from *E. coli,* the phosphatase yqaB from *E. coli* with SEQ ID NO 19, galE from *E. coli* with SEQ ID NO 10, LgtA from *N. meningitidis* with SEQ ID NO 07, LgtB with SEQ ID NO 09 from *N. meningitidis,* WbpP from *P. aeruginosa* with SEQ ID NO 14 and LgtD from *H. influenzae* with SEQ ID NO 15. When evaluated on SD CSM medium comprising lactose, the mutant yeast strain produces an oligosaccharide mixture comprising LacNAc, LN3, LNnT, GaINAc-b1,3-lactose, Gal-b1,3-GalNAc-b1,3-lactose, GalNAc-b1,3-Gal-b1,4-GlcNAc-b1,3-Gal-b1,4-Glc, GalNAc-b1,3-LacNAc, Gal-b1,3-GaINAc-b1,3-LacNAc, poly-LacNAc structures, i.e. (Gal-b1,4-GlcNAc)n and GalNAc-ylated poly-LacNAc structures.

### Example 20. Production of an oligosaccharide mixture comprising LNB, LN3, LNT, GaINAc-b1,3-lactose, Gal-b1,3- GalNAc-b1,3-lactose, GalNAc-b1,3-LNB and Gal-b1,3-GalNAc-b1,3-LNB with a modified S. cerevisiae host

An S. *cerevisiae* strain was transformed with a YAC comprising constitutive transcriptional units for the mutant glmS*54 with SEQ ID NO 06 from *E. coli,* the phosphatase yqaB from *E. coli* with SEQ ID NO 19, WbgO with SEQ ID NO 08 from *E. coli* O55:H7, WbpP of *P. aeruginosa* with SEQ ID NO 14, LgtA from N. *meningitidis* with SEQ ID NO 07 and LgtD from *H. influenzae* with SEQ ID NO 15. When evaluated on SD CSM medium comprising lactose, the mutant yeast strain produces an oligosaccharide mixture comprising LNB, LN3, LNT, GaINAc-b1,3-lactose, Gal-b1,3-GalNAc-b1,3-lactose, GalNAc-b1,3-Gal-b1,3-GlcNAc-b1,3-Gal-b1,4-Glc, GalNAc-b1,3-LNB and Gal-b1,3-Gal NAc-b1,3-LN B.

### Example 21. Production of an oligosaccharide mixture comprising galactosylated and GaINAc-ylated lactose structures with a modified S. cerevisiae host

An S. *cerevisiae* strain was transformed with a YAC comprising constitutive transcriptional units for the mutant glmS*54 with SEQ ID NO 06 from *E. coli,* the phosphatase yqaB from *E. coli* with SEQ ID NO 19, galE from *E. coli* with SEQ ID NO 10, WbpP of *P*. *aeruginosa* with SEQ ID NO 14, LgtA from *N. meningitidis* with SEQ ID NO 07 and LgtD from *H. influenzae* with SEQ ID NO 15, the a1,4-galactosyltransferase (LgtC) from *N. gonorrhoeae* with SEQ ID NO 16. When evaluated on SD CSM medium comprising lactose, the mutant yeast strain produces an oligosaccharide mixture comprising Gal-a1,4-Gal-b1,4-Glc (Gal-a1,4-lactose), Gal-a1,4-Gal-a1,4-Gal-b1,4-Glc, Gal-a1,4-Gal-a1,4-Gal-a1,4-Gal-b1,4-Glc, GalNAc-b1,3-Gal-b1,4-Glc (GaINAc-b1,3-Lactose), Gal-b1,3-GaINAc-b1,3-lactose, GaINAc-b1,3-Gal-a1,4-Gal-b1,4-Glc (globo-N-tetraose) and Gal-b1,3-GaINAc-b1,3-Gal-a1,4-Gal-b1,4-Glc.

### Example 22. Production of an oligosaccharide mixture comprising LN3, LNT, GaINAc-b1,3-LNT, Gal-b1,3-GaINAc-b1,3-LNT, GaINAc-b1,3-lactose and Gal-b1,3-GaINAc-b1,3-lactose with a modified S. cerevisiae host

An S. *cerevisiae* strain was transformed with a YAC comprising constitutive transcriptional units for the mutant glmS^{∗}54 with SEQ ID NO 06 from *E. coli,* LgtA from *N. meningitidis* with SEQ ID NO 07, WbgO from *E. coli* O55:H7 with SEQ ID NO 08, WbpP from *P. aeruginosa* with SEQ ID NO 14 and LgtD from *H. influenzae* with SEQ ID NO 15. When evaluated on SD CSM medium comprising lactose, the mutant yeast strain produces an oligosaccharide mixture comprising LN3, LNT, GaINAc-b1,3-LNT, Gal-b1,3-GaINAc-b1,3-LNT, GaINAc-b1,3-lactose and Ga I-b 1, 3-Ga I NAc-b 1, 3-lactose.

### Example 23. Production an oligosaccharide mixture comprising LN3, LNnT, GalNAc-b1,3-LNnT, Gal-b1,3-GaINAc-b1,3-LNnT, GaINAc-b1,3-lactose, Gal-b1,3-GaINAc-b1,3-lactose and (GaINAc)-poly-LNnT structures with a modified S. cerevisiae host

An S. *cerevisiae* strain was transformed with a YAC comprising constitutive transcriptional units for the mutant glmS*54 with SEQ ID NO 06 from *E. coli,* LgtA from *N. meningitidis* with SEQ ID NO 07, LgtB with SEQ ID NO 09 from *N. meningitidis,* WbpP from *P. aeruginosa* with SEQ ID NO 14 and LgtD from *H*. *influenzae* with SEQ ID NO 15. When evaluated on SD CSM medium comprising lactose, the mutant yeast strain produces an oligosaccharide mixture comprising LN3, LNnT, GaINAc-b1,3-LNnT, Gal-b1,3-GalNAc-b1,3-LNnT, GaINAc-b1,3-lactose and Gal-b1,3-GaINAc-b1,3-lactose as well as poly-LNnT structures and GalNAc-ylated poly-LNnT structures.

## Claims

1. A metabolically engineered cell producing a mixture of at least four different neutral non-fucosylated oligosaccharides, wherein said cell
- expresses at least three glycosyltransferases, and
- is capable to synthesize one or more nucleotide-sugar(s), wherein said nucleotide-sugar(s) is/are donor(s) for said glycosyltransferases.

2. Cell according to claim 1, wherein said cell is genetically engineered for the production of said oligosaccharides.

3. Cell according to claim 1 or 2, wherein said cell is modified with gene expression modules, **characterized in that** the expression from any of said expression modules is either constitutive or is created by a natural inducer.

4. Cell according to any one of claims 1 to 3, wherein said oligosaccharide mixture comprises at least three different oligosaccharides differing in degree of polymerization.

5. Cell according to any one of claims 1 to 4, wherein any one of said glycosyltransferase is chosen from the list comprising galactosyltransferases, glucosyltransferases, mannosyltransferases, N-acetylglucosaminyltransferases, N-acetylgalactosaminyltransferases, N-acetylmannosaminyltransferases, xylosyltransferases, glucosaminyltransferases, rhamnosyltransferases, preferably said cell is modified in the expression or activity of at least one of said glycosyltransferases.

6. Cell according to any one of claims 1 to 5, wherein any one of said nucleotide-sugar is chosen from the list comprising UDP-N-acetylglucosamine (UDP-GIcNAc), UDP-galactose (UDP-Gal), UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, UDP-xylose.

7. Cell according to any one of claims 1 to 6, wherein said mixture comprises at least one neutral non-fucosylated oligosaccharide that is galactosylated, glucosylated, xylosylated, mannosylated, contains an N-acetylglucosamine, contains an N-acetylgalactosamine, contains a rhamnose, and/or contains an N-acetylmannosamine.

8. A cell according to any one of claims 1 to 7, wherein any one, preferably all, of said neutral non-fucosylated oligosaccharides is/are (a) mammalian milk oligosaccharide(s).

9. A method to produce a mixture of at least four different neutral non-fucosylated oligosaccharides by a cell, the method comprising the steps of:
a. providing a cell expressing at least three glycosyltransferases and capable to synthesize one or more nucleotide-sugar(s), wherein said nucleotide-sugar(s) is/are donor(s) for said glycosyltransferases,
b. cultivating said cell under conditions permissive to express said glycosyltransferases and to synthesize said nucleotide-sugars,
c. preferably, separating at least one of said neutral non-fucosylated oligosaccharides from said cultivation.

10. Method according to claim 9, wherein said cell is a metabolically engineered cell according to any one of claims 1 to 8.

11. Method according to claim 9 or 10, wherein any one of said glycosyltransferases is chosen from the list comprising galactosyltransferases, glucosyltransferases, mannosyltransferases, N-acetylglucosaminyltransferases, N-acetylgalactosaminyltransferases, N-acetylmannosaminyltransferases, xylosyltransferases, glucosaminyltransferases, rhamnosyltransferases.

12. Method according to any one of claim 9 to 11, wherein any one of said nucleotide-sugars is chosen from the list comprising UDP-GIcNAc, UDP-Gal, UDP-N-acetylgalactosamine (UDP-GaINAc), UDP-N-acetylmannosamine (UDP-ManNAc), GDP-mannose (GDP-Man), UDP-glucose (UDP-Glc), GDP-rhamnose, UDP-xylose.

13. Method according to any one of claims 9 to 12, wherein any one of said neutral non-fucosylated oligosaccharides is galactosylated, glucosylated, xylosylated, mannosylated, contains an N-acetylglucosamine, contains an N-acetylgalactosamine, contains a rhamnose, and/or contains an N-acetylmannosamine.

14. Method according to any one of claim 9 to 13, wherein said cell uses at least one precursor for synthesis of any one or more of said oligosaccharides, preferably said cell uses two or more precursors for the synthesis of any one or more of said oligosaccharides.

15. Use of a cell according to any one of claims 1 to 8, or method according to any one of claim 9 to 14 for the production of a mixture of at least four different neutral non-fucosylated oligosaccharides.

## Patentansprüche

1. Metabolisch veränderte Zelle, die ein Gemisch aus mindestens vier verschiedenen neutralen, nicht fucosylierten Oligosacchariden erzeugt, wobei die Zelle mindestens drei Glycosyltransferasen exprimiert, und
in der Lage ist, einen oder mehrere Nukleotidzucker zu synthetisieren, wobei der (die) Nukleotidzucker Donor(en) für die Glycosyltransferasen ist/sind.

2. Zelle nach Anspruch 1, wobei die Zelle für die Erzeugung der Oligosaccharide gentechnisch verändert ist.

3. Zelle nach Anspruch 1 oder 2, wobei die Zelle mit Genexpressionsmodulen modifiziert ist, **dadurch gekennzeichnet, dass** die Expression von einem der Expressionsmodule entweder konstitutiv ist oder durch einen natürlichen Induktor erzeugt wird.

4. Zelle nach einem der Ansprüche 1 bis 3, wobei das Oligosaccharidgemisch mindestens drei verschiedene Oligosaccharide mit unterschiedlichem Polymerisationsgrad umfasst.

5. Zelle nach einem der Ansprüche 1 bis 4, wobei eine der Glycosyltransferasen ausgewählt ist aus der Liste, umfassend Galactosyltransferasen, Glucosyltransferasen, Mannosyltransferasen, N-Acetylglucosaminyltransferasen, N-Acetylgalactosaminyltransferasen, N-Acetylmannosaminyltransferasen, Xylosyltransferasen, Glucosaminyltransferasen, Rhamnosyltransferasen, vorzugsweise wobei die Zelle in Bezug auf die Expression oder Aktivität von mindestens einer der Glycosyltransferasen modifiziert ist.

6. Zelle nach einem der Ansprüche 1 bis 5, wobei einer der Nukleotidzucker ausgewählt ist aus der Liste, umfassend UDP-N-Acetylglucosamin (UDP-GIcNAc), UDP-Galactose (UDP-Gal), UDP-N-Acetylgalactosamin (UDP-GalNAc), UDP-N-Acetylmannosamin (UDP-ManNAc), GDP-Mannose (GDP-Man), UDP-Glucose (UDP-Glc), GDP-Rhamnose, UDP-Xylose.

7. Zelle nach einem der Ansprüche 1 bis 6, wobei das Gemisch mindestens ein neutrales, nicht fucosyliertes Oligosaccharid umfasst, das galactosyliert, glucosyliert, xylosyliert, mannosyliert ist, ein N-Acetylglucosamin enthält, ein N-Acetylgalactosamin enthält, eine Rhamnose enthält und/oder ein N-Acetylmannosamin enthält.

8. Zelle nach einem der Ansprüche 1 bis 7, wobei eines, vorzugsweise alle, der neutralen, nicht fucosylierten Oligosaccharide (ein) Säugermilch-Oligosaccharid(e) ist/sind.

9. Verfahren zum Erzeugen eines Gemischs aus mindestens vier verschiedenen neutralen nicht-fucosylierten Oligosacchariden durch eine Zelle, das Verfahren umfassend die folgenden Schritte:
a. Bereitstellen einer Zelle, die mindestens drei Glycosyltransferasen exprimiert und in der Lage ist, einen oder mehrere Nukleotidzucker zu synthetisieren, wobei der (die) Nukleotidzucker Donor(en) für die Glycosyltransferasen ist/sind,
b. Kultivieren der Zelle unter Bedingungen, die die Expression der Glycosyltransferasen und die Synthese der Nukleotidzucker ermöglichen,
c. vorzugsweise Abscheiden mindestens eines der neutralen, nicht fucosylierten Oligosaccharide aus der Kultivierung.

10. Verfahren nach Anspruch 9, wobei die Zelle eine metabolisch veränderte Zelle nach einem der Ansprüche 1 bis 8 ist.

11. Verfahren nach Anspruch 9 oder 10, wobei eine der Glycosyltransferasen ausgewählt ist aus der Liste, umfassend Galactosyltransferasen, Glucosyltransferasen, Mannosyltransferasen, N-Acetylglucosaminyltransferasen, N-Acetylgalactosaminyltransferasen, N-Acetylmannosaminyltransferasen, Xylosyltransferasen, Glucosaminyltransferasen, Rhamnosyltransferasen.

12. Verfahren nach einem der Ansprüche 9 bis 11, wobei einer der Nukleotidzucker ausgewählt ist aus der Liste, umfassend UDP-GIcNAc, UDP-Gal, UDP-N-Acetylgalactosamin (UDP-GalNAc), UDP-N-Acetylmannosamin (UDP-ManNAc), GDP-Mannose (GDP-Man), UDP-Glucose (UDP-Glc), GDP-Rhamnose, UDP-Xylose.

13. Verfahren nach einem der Ansprüche 9 bis 12, wobei eines der neutralen, nicht fucosylierten Oligosaccharide galactosyliert, glucosyliert, xylosyliert, mannosyliert ist, ein N-Acetylglucosamin enthält, ein N-Acetylgalactosamin enthält, eine Rhamnose enthält und/oder ein N-Acetylmannosamin enthält.

14. Verfahren nach einem der Ansprüche 9 bis 13, wobei die Zelle mindestens einen Vorläufer für die Synthese von einem oder mehreren der Oligosaccharide verwendet, vorzugsweise wobei die Zelle zwei oder mehr Vorläufer für die Synthese von einem oder mehreren der Oligosaccharide verwendet.

15. Verwendung einer Zelle nach einem der Ansprüche 1 bis 8 oder Verfahren nach einem der Ansprüche 9 bis 14 zum Erzeugen eines Gemischs aus mindestens vier verschiedenen neutralen nicht-fucosylierten Oligosacchariden.

## Revendications

1. Cellule métaboliquement modifiée produisant un mélange d'au moins quatre oligosaccharides non fucosylés neutres différents, ladite cellule
- exprimant au moins trois glycosyltransférases, et
- étant capable de synthétiser un ou plusieurs sucres nucléotidiques, ledit ou lesdits sucres nucléotidiques étant un/des donneur(s) pour lesdites glycosyltransférases.

2. Cellule selon la revendication 1, ladite cellule étant génétiquement modifiée pour la production desdits oligosaccharides.

3. Cellule selon la revendication 1 ou 2, ladite cellule étant modifiée par des modules d'expression génique, **caractérisée en ce que** l'expression à partir de l'un quelconques desdits modules d'expression est soit constitutive, soit créée par un inducteur naturel.

4. Cellule selon l'une quelconque des revendications 1 à 3, ledit mélange d'oligosaccharides comprenant au moins trois oligosaccharides différents différant en degré de polymérisation.

5. Cellule selon l'une quelconque des revendications 1 à 4, l'une quelconque desdites glycosyltransférases étant choisie dans la liste comprenant des galactosyltransférases, des glucosyltransférases, des mannosyltransférases, des N-acétylglucosaminyltransférases, des N-acétylgalactosaminyltransférases, des N-acétylmannosaminyltransférases, des xylosyltransférases, des glucosaminyltransférases, des rhamnosyltransférases, préférablement ladite cellule étant modifiée dans l'expression ou l'activité d'au moins l'une desdites glycosyltransférases.

6. Cellule selon l'une quelconque des revendications 1 à 5, l'un quelconque desdits sucres nucléotidiques étant choisi dans la liste comprenant UDP-N-acétylglucosamine (UDP-GlcNAc), UDP-galactose (UDP-Gal), UDP-N-acétylgalactosamine (UDP-GalNAc), UDP-N-acétylmannosamine (UDP-ManNAc), GDP-mannose (GDP-Man), UDP-glucose (UDP-Glc), GDP-rhamnose, UDP-xylose.

7. Cellule selon l'une quelconque des revendications 1 à 6, ledit mélange comprenant au moins un oligosaccharide non fucosylé neutre qui est galactosylé, glucosylé, xylosylé, mannosylé, contient une N-acétylglucosamine, contient une N-acétylgalactosamine, contient un rhamnose, et/ou contient une N-acétylmannosamine.

8. Cellule selon l'une quelconque des revendications 1 à 7, l'un quelconque, préférablement la totalité, desdits oligosaccharides non fucosylés neutres étant un/des oligosaccharide(s) de lait de mammifère.

9. Procédé de production d'un mélange d'au moins quatre oligosaccharides non fucosylés neutres différents par une cellule, le procédé comprenant les étapes de :
a. fourniture d'une cellule exprimant au moins trois glycosyltransférases et capable de synthétiser un ou plusieurs sucres nucléotidiques, ledit ou lesdits sucres nucléotidiques étant un/des donneur(s) pour lesdites glycosyltransférases,
b. culture de ladite cellule dans des conditions permissives pour exprimer lesdites glycosyltransférases et pour synthétiser lesdits sucres nucléotidiques,
c. préférablement, séparation d'au moins l'un desdits oligosaccharides non fucosylés neutres de ladite culture.

10. Procédé selon la revendication 9, ladite cellule étant une cellule métaboliquement modifiée selon l'une quelconque des revendications 1 à 8.

11. Procédé selon la revendication 9 ou 10, l'une quelconque desdites glycosyltransférases étant choisie dans la liste comprenant des galactosyltransférases, des glucosyltransférases, des mannosyltransférases, des N-acétylglucosaminyltransférases, des N-acétylgalactosaminyltransférases, des N-acétylmannosaminyltransférases, des xylosyltransférases, des glucosaminyltransférases, des rhamnosyltransférases.

12. Procédé selon l'une quelconque des revendications 9 à 11, l'un quelconque desdits sucres nucléotidiques étant choisi dans la liste comprenant UDP-GlcNAc, UDP-Gal, UDP-N-acétylgalactosamine (UDP-GalNAc), UDP-N-acétylmannosamine (UDP-ManNAc), GDP-mannose (GDP-Man), UDP-glucose (UDP-Glc), GDP-rhamnose, UDP-xylose.

13. Procédé selon l'une quelconque des revendications 9 à 12, l'un quelconque desdits oligosaccharides non fucosylés neutres étant galactosylé, glucosylé, xylosylé, mannosylé, contenant une N-acétylglucosamine, contenant une N-acétylgalactosamine, contenant un rhamnose, et/ou contenant une N-acétylmannosamine.

14. Procédé selon l'une quelconque des revendications 9 à 13, ladite cellule utilisant au moins un précurseur pour la synthèse de l'un quelconque ou plusieurs desdits oligosaccharides, préférablement ladite cellule utilisant deux précurseurs ou plus pour la synthèse de l'un quelconques ou plusieurs desdits oligosaccharides.

15. Utilisation d'une cellule selon l'une quelconque des revendications 1 à 8, ou procédé selon l'une quelconque des revendications 9 à 14 pour la production d'un mélange d'au moins quatre oligosaccharides non fucosylés neutres différents.
